# EUROPEAN PATENT SPECIFICATION

(11) **EP 3 981 469 B1**
(45) Date of publication and mention of the grant of the patent: **20.11.2024**
(21) Application number: 20817851.7
(22) Date of filing: 04.06.2020
(51) Int. Cl.: A61K 31/519, A61P 19/06, A61K 9/48, A61K 47/38, C07D 513/04, A61K 9/16

(54) **THERAPEUTIC FOR GOUT OR HYPERURICEMIA**
THERAPEUTIKUM FÜR GICHT ODER HYPERURIKÄMIE
AGENT THÉRAPEUTIQUE CONTRE LA GOUTTE OU L'HYPERURICÉMIE

(30) Priority: 04.06.2019 JP 2019104534
(43) Date of publication of application: 13.04.2022
(73) Proprietor: Nippon Chemiphar Co., Ltd., Tokyo 101-0032 (JP)
(72) Inventor: KOBAYASHI Shiro, Tokyo 101-0032 (JP); MIYAYAMA Eri, Tokyo 101-0032 (JP); HIRANO Masuharu, Misato-shi, Saitama 341-0005 (JP); OHTA Takashi, Misato-shi, Saitama 341-0005 (JP)
(74) Representative: V.O.
(86) International application number: PCT/JP2020/022039
(87) International publication number: WO 2020/246528

(56) References cited:
- EP-A1- 1 757 610
- WO-A1-2005/121153
- WO-A1-2019/107412
- JP-A- 2016 520 133
- TAMBOSI GABRIELA ET AL: "Challenges to improve the biopharmaceutical properties of poorly water-soluble drugs and the application of the solid dispersion technology", MATÉRIA (RIO DE JANEIRO), vol. 23, no. 4, 6 December 2018 (2018-12-06), XP093044326, DOI: 10.1590/s1517-707620180004.0558
- KHANNA DINESH ET AL: "2012 American College of Rheumatology guidelines for management of gout. Part 1: Systematic nonpharmacologic and pharmacologic therapeutic approaches to hyperuricemia", ARTHRITIS CARE RESEARCH, vol. 64, no. 10, 1 October 2012 (2012-10-01), US, pages 1431 - 1446, XP055804638, ISSN: 2151-464X, Retrieved from the Internet <URL:https://www.ncbi.nlm.nih.gov/pmc/articles/PMC3683400/pdf/nihms416576.pdf> DOI: 10.1002/acr.21772
- KASHIHARA TOSHIO: "Chapter 4 Strategies for design and development of oral dosage forms", DESIGN AND EVALUATION OF ORAL DOSAGE FORMS, vol. 4, 1995, pages 47 - 67, XP009532819
- WATARU KAWAKAMI: "Leading Edge Technologies for Oral Delivery of Poorly Soluble Drugs", 6 July 2016, CMC PUBLISHING, JP, ISBN: 978-4-7813-1169-2, article ONOUE SATOMI: "Chapter 3.1 Current status of drug development using amorphous technology", pages: 127 - 135, XP009532824
- TANNO, FUMIE: "Evaluation of Characteristics of Hypromellose Acetate Succinate (HPMCAS) as a Carrier for Solid Dispersion -from Study to Improve In Vitro Dissolution of Poorly Water Soluble Drugs", JOURNAL OF PHARMACEUTICAL SCIENCE AND TECHNOLOGY, JAPAN, vol. 73, no. 4, 2013, pages 214 - 222, XP009520813, DOI: 10.14843/jpstj.73.214

## Description

### Technical Field

The present invention relates to a pharmaceutical composition for treating or preventing gout or hyperuricemia.

Priority is claimed on Japanese Patent Application No. 2019-104534, filed June 4, 2019.

The present invention is defined by the claims. Any subject-matter falling outside the scope of the claims is provided for information only.

Methods of treatment of the human body by therapy, referred to in this description, are not part of the present invention as such, but are described here in relation to compounds and pharmaceutical compositions for use in said methods for treatment of the human body by therapy, according to the present invention.

### Background Art

Hyperuricemia causes gout, kidney failure, and the like, and is considered to be a risk factor for coronary artery disease. In addition, it has been pointed out that hyperuricemia has a close relationship with the onset and progression of lifestyle-related diseases such as hypertension. Therefore, the treatment of hyperuricemia includes not only treatment of gout but also prevention of various lifestyle-related diseases associated with aging.

Currently, xanthine oxidase inhibitors such as allopurinol and febuxostat are mainly used for the treatment of hyperuricemia.

These drugs are very excellent from the viewpoint of lowering a uric acid level and have been used for many years.

Meanwhile, in the treatment of hyperuricemia, how to suppress a gout attack is problematic. A gout attack is caused when uric acid becomes excessive in blood, and strong inflammation occurs due to accumulation of crystals of the uric acid in the joints, and is accompanied by severe pain.

Here, it is known that the uric acid-lowering drugs and an acute gout attack are related, and a rapid decrease in serum uric acid results in transient localized precipitation of monosodium urate crystals in cartilage and soft tissue, leading to the acute gout attack. That is, it is known that the conventional uric acid-lowering drugs can induce a gout attack. Therefore, depending on the patient, it may be necessary to discontinue the treatment or change the treatment strategy due to the gout attack (Non Patent Literature 1).

It has been reported that, in order to cope with the above-mentioned problem, allopurinol has been used in combination with colchicine during the first 6 months of the administration as a preventive measure against gout attacks, and such combined usage resulted in reduction in the incidence of gout attacks (Non Patent Literature 2). However, colchicine is limited in use and contraindicated in patients with a liver or kidney disorder. Colchicine has also been reported to have side effects.

Regarding febuxostat, in order to cope with the above-mentioned problem, a usual adult dose is started at a low dose of 10 mg once daily, and then the dose is increased in a stepwise manner to 20 mg once daily and then to a usual maintenance dose of 40 mg once daily, thereby gradually lowering the uric acid level to reduce the incidence of the attacks. In addition, the gout guideline states that the uric acid level is to be lowered over 3 to 6 months, since a rapid decrease in the uric acid level may induce a gout attack.

Patent Literature 1 discloses a method of preventing at least one gout attack or reducing the number of gout attacks by administering an effective dose of a xanthine oxidase inhibitor to a patient with hyperuricemia once daily in a modified release administration form or twice or more daily in an immediate release administration form.

A compound described in WO 2005/121153 A (Patent Literature 2) has been reported as a compound having a similar mechanism as those of the above drugs.

Patent Literature 2 discloses a comparative experiment on plasma uric acid level-suppressing rates of a compound of the invention and the allopurinol or febuxostat in Pharmacological Experiment 2 of Example 51, and describes that the compound of the invention exhibits an equivalent or higher xanthine oxidase inhibitory action compared to the control allopurinol or febuxostat.

### Citation List

### Patent Literature

Patent Literature 1: JP 2016-520133 A
Patent Literature 2: WO 2005/121153 A

### Non Patent Literature

Non Patent Literature 1: Arthritis Res Ther. 2009; 11: R46
Non Patent Literature 2: J Rheumatol 2004; 31; 2429-2432
Non Patent Literature 3: Application Summary of Feburic (Registered Trademark) Tablet [January 2011]; 2.7.2 Clinical Pharmacology Study, P.30

### Summary of Invention

### Technical Problem

As a result of performing studies on treatment of gout or hyperuricemia which reduces the induction of a gout attack or severity of an attack without using a drug having a xanthine oxidase inhibitory activity in combination with colchicine and without increasing a dose of the drug having a xanthine oxidase inhibitory activity in a stepwise manner from a low dose, the present inventors found that there is still room for improvement in the treatment of gout or hyperuricemia with a drug having a xanthine oxidase inhibitory activity.

An object of the present invention is to provide a pharmaceutical composition for treating or preventing gout or hyperuricemia. An object of the present invention is to provide a pharmaceutical composition for treating or preventing gout or hyperuricemia which reduces the induction of a gout attack or severity of an attack without using a drug having a xanthine oxidase inhibitory activity in combination with colchicine and without increasing a dose of the drug having a xanthine oxidase inhibitory activity in a stepwise manner from a low dose.

### Solution to Problem

As a result of performing various studies on a drug having a xanthine oxidase inhibitory activity, the present inventors found that continuous administration of a certain amount of the pharmaceutical composition containing a compound represented by General Formula (I) or a pharmaceutically acceptable salt thereof is effective in the treatment of gout or hyperuricemia which suppresses the induction of a gout attack or reduces severity of an attack without using the pharmaceutical composition in combination with colchicine and without gradually increasing the dose of the pharmaceutical composition, and the present invention was completed based on this finding.

That is, the present invention relates to the following.
[1] A pharmaceutical composition for use in treating gout or hyperuricemia, or hyperuricemia in a gouty arthritis patient, the pharmaceutical composition comprising:
   a compound represented by General Formula (I): 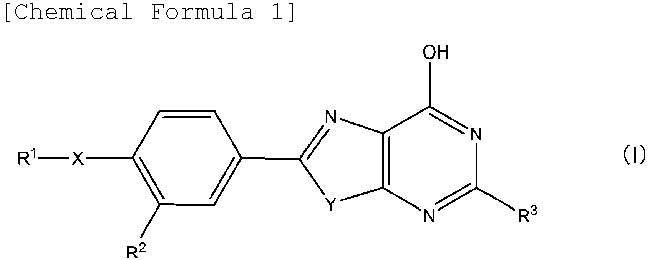 wherein R¹ is an unsubstituted phenyl group or a phenyl group substituted with a substituent, the substituent being at least one group selected from the group consisting of an alkyl group having 1 to 8 carbon atoms, an alkyl group having 1 to 8 carbon atoms substituted with a halogen atom, an alkoxy group having 1 to 8 carbon atoms, an alkoxycarbonyl group having 2 to 8 carbon atoms, a formyl group, a carboxyl group, a halogen atom, a phenyl group, and a phenoxy group, R² is a cyano group or a nitro group, R³ is a hydrogen atom or a hydroxyl group, X is an oxygen atom or -S(O)ₙ-, n is an integer of 0 to 2, and Y is an oxygen atom or a sulfur atom, or a pharmaceutically acceptable salt thereof, wherein the content of the compound represented by General Formula (I) or a pharmaceutically acceptable salt thereof is 10 mg to 320 mg, the oral administration thereof is continued for at least 7 days, and the patient is a human.
[2] The pharmaceutical composition for use according to [1], which suppresses onset of a gout attack accompanying initiation of a uric acid-lowering therapy thereby.
[3] The pharmaceutical composition for use according to [1] or [2], which is an enteric-coated preparation.
[4] The pharmaceutical composition for use according to [3], wherein the enteric-coated preparation is a hard capsule.
[5] The pharmaceutical composition for use according to any one of [1] to [4], further comprising: a solid dispersion containing a hypromellose, or an organic acid ester of hypromellose.
[6] The pharmaceutical composition for use according to [5], wherein the organic acid ester of hypromellose is hypromellose acetate succinate or hypromellose phthalate.
[7] The pharmaceutical composition for use according to [5] or [6], wherein a weight ratio between the compound represented by General Formula (I) or a pharmaceutically acceptable salt thereof and the hypromellose or the organic acid ester of hypromellose is 1:0.1 to 1:25.
[8] The pharmaceutical composition for use according to any one of [1] to [7], which is a solid preparation.
[9] The pharmaceutical composition for use according to any one of [1] to [8], wherein a content of the compound represented by General Formula (I) or a pharmaceutically acceptable salt thereof per dosage unit is 10 mg to 320 mg, 10 mg to 160 mg, 10 mg to 80 mg, or 20 mg to 80 mg.
[10] The pharmaceutical composition for use according to [9], wherein a blood uric acid concentration is lowered by 0.5 to 1.5 mg/dL at 12 hours after administration on the first day of the administration as compared with a blood uric acid concentration before the administration; or by continuous administration once/ day for 7 days, a blood uric acid concentration is lowered by 1.5 to 3.0 mg/dL at 12 hours after administration on the seventh day of the administration as compared with the blood uric acid concentration before the administration.
[11] The pharmaceutical composition for use according to any one of [1] to [10], wherein a daily dose of the compound represented by General Formula (I) or a pharmaceutically acceptable salt thereof is not increased within 3 weeks of the administration after the start of the administration; or is not increased or is increased once within 7 weeks of the administration after the start of the administration.
[12] The pharmaceutical composition for use according to [11], wherein a maximum rate of decrease in a blood uric acid level on the first day of the administration, which is calculated by the following formula: [(pre-administration uric acid level - minimum post-administration uric acid level on first day of administration)/pre-administration uric acid level] x100, is 10 to 250; or maximum rate of decrease in a blood uric acid level on the seventh day of the administration, which is calculated by the following formula: [(pre-administration uric acid level - minimum post-administration uric acid level on the seventh day of administration)/pre-administration uric acid level] x 100, is 20 to 45%.
[13] The pharmaceutical composition for use according to any one of [1] to [12], wherein the compound represented by General Formula (I) or a pharmaceutically acceptable salt thereof is 2-(3-cyano-4-phenoxyphenyl)-7-hydroxythiazolo[5,4-d]pyrimidine or a pharmaceutically acceptable salt thereof.
[14] A package comprising: the pharmaceutical composition for use according to any one of [1] to [13], wherein the number of dosage units of the pharmaceutical composition in the package is the number required for continuous administration for 5 to 15 days.

### Advantageous Effects of Invention

The pharmaceutical composition provided by the present invention is useful for the treatment of gout or hyperuricemia.

### Brief Description of Drawings

Fig. 1 is a diagram showing a particle size distribution of amorphous Compound 14 measured by a wet laser diffraction method.
Fig. 2 is a diagram showing plasma drug concentration-time curves when a capsule of Reference Example 1a and a capsule of Comparative Reference Example 1a were administered to dogs.
Fig. 3 is a diagram showing a powder X-ray diffraction pattern of crystalline Compound 14.

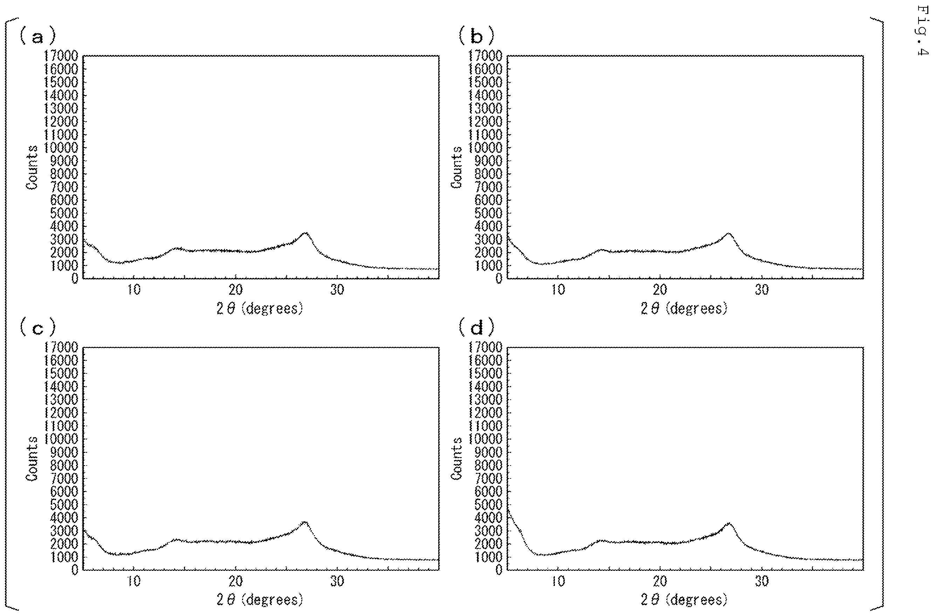

Fig. 4 is a diagram showing powder X-ray diffraction patterns of amorphous Compound 14, in which (a) is a powder X-ray diffraction pattern before storage, (b) is a powder X-ray diffraction pattern after storage for 1 week under a room temperature condition in a light-shielded and airtight manner, (c) is a powder X-ray diffraction pattern after storage for 2 weeks under a room temperature condition in a light-shielded and airtight manner, and (d) is a powder X-ray diffraction pattern after storage after storage for 4 weeks under a room temperature condition in a light-shielded and airtight manner.
Fig. 5 is a diagram showing powder X-ray diffraction patterns of a solid dispersion of Reference Example 9b before storage and after 1 week, 3 weeks, and 7 weeks of storage under open conditions at 40 °C/75% RH, respectively.
Fig. 6 is a diagram showing powder X-ray diffraction patterns of a solid dispersion of Reference Example 10b before storage and after 1 week, 3 weeks, and 7 weeks of storage under open conditions at 40 °C/75% RH, respectively.
Fig. 7 is a diagram showing powder X-ray diffraction patterns of a solid dispersion of Reference Example 11b before storage and after 1 week, 3 weeks, and 7 weeks of storage under open conditions at 40 °C/75% RH, respectively.
Fig. 8 is a graph showing an action of a solid dispersion of Reference Example 11b lowering a plasma uric acid level in rats.
Fig. 9 is a diagram showing decreases in serum uric acid levels when Compound 14 and, as a control drug, febuxostat were orally administered to healthy adult males in Example 1.

### Description of Embodiments

### <Pharmaceutical composition>

The present invention will be described below in further detail. A pharmaceutical composition of the present invention for treating or preventing gout or hyperuricemia includes a compound represented by General Formula (I) or a pharmaceutically acceptable salt thereof as an active ingredient.

In a compound represented by General Formula (I), R¹ is an unsubstituted phenyl group or a phenyl group substituted with a substituent.

Examples of an "alkyl group having 1 to 8 carbon atoms" as a substituent in the phenyl group represented by R¹ include a methyl group, an ethyl group, a propyl group, an isopropyl group, a butyl group, an isobutyl group, a tert-butyl group, a pentyl group, and a hexyl group, and a methyl group or an ethyl group is preferable.

Examples of an "alkyl group having 1 to 8 carbon atoms substituted with a halogen atom" as a substituent in the phenyl group represented by R¹ include a fluoromethyl group, a trifluoromethyl group, a 1,1-difluoroethyl group, and a pentafluoroethyl group, and a fluoromethyl group or a trifluoromethyl group is preferable.

Examples of an "alkoxy group having 1 to 8 carbon atoms" as a substituent in the phenyl group represented by R¹ include a methoxy group, an ethoxy group, a propoxy group, an isopropoxy group, a butoxy group, an isobutoxy group, and a tert-butoxy group, and a methoxy group is preferable.

Examples of an "alkoxycarbonyl group having 2 to 8 carbon atoms" as a substituent in the phenyl group represented by R¹ include a methoxycarbonyl group, an ethoxycarbonyl group, a propoxycarbonyl group, a butoxycarbonyl group, and a tert-butoxycarbonyl group, and a methoxycarbonyl group or an ethoxycarbonyl group is preferable.

Examples of a "halogen atom" as a substituent in the phenyl group represented by R¹ include a fluorine atom, a chlorine atom, a bromine atom, and an iodine atom, and a fluorine atom or a chlorine atom is preferable.

Regarding R¹, an unsubstituted phenyl group is preferable.

In the compound represented by General Formula (I), R² is a cyano group or a nitro group, and a cyano group is preferable.

In the compound represented by General Formula (I), R³ represents a hydrogen atom or a hydroxy group, and a hydrogen atom is preferable.

In the compound represented by General Formula (I), X is an oxygen atom or -S(O)ₙ-, and an oxygen atom is preferable.

In the compound represented by General Formula (I), Y is an oxygen atom or a sulfur atom, and a sulfur atom is preferable.

Examples of the pharmaceutically acceptable salt of the compound represented by General Formula (I) include an alkali metal salt such as a sodium salt, a potassium salt, and a lithium salt, and a potassium salt is preferable.

The compound of General Formula (I) included in the pharmaceutical composition for treating or preventing gout or hyperuricemia, which is one embodiment of the present invention, can be obtained by, for example, the synthesis method described in Patent Literature 2 or PCT/JP 2019/17439.

As the compound of General Formula (I) included in the pharmaceutical composition of the present invention for treating or preventing gout or hyperuricemia, the compounds of Table 1 are preferable. In the table, Me is a methyl group.

**[Table 1]**

| | Structural formula | Compound name |
|---|---|---|
| Compound 1 | 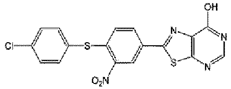 | 2-[4-(4-Chlorophenylthio)-3-nitrophenyl]-7-hydroxythiazolo[5,4-d]pyrimidine |
| Compound 2 | 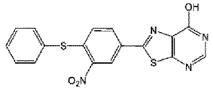 | 7-Hydroxy-2-(3-nitro-4-phenylthiophenyl)thiazolo[5,4-d]pyrimidine |
| Compound 3 | 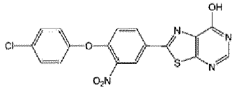 | 2-[4-(4-Chlorophenoxy)-3-nitrophenyl]-7-hydroxythiazolo[5,4-d]pyrimidine |
| Compound 4 | 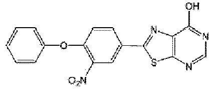 | 7-Hydroxy-2-(3-nitro-4-phenoxyphenyl)thiazolo[5,4-d]pyrimidine |
| Compound 5 | 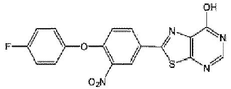 | 2-[4-(4-Fluorophenoxy)-3-nitrophenyl]-7-hydroxythiazolo[5,4-d]pyrimidine |
| Compound 6 | 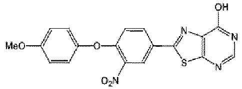 | 2-[4-(4-Methoxyphenoxy)-3-nitrophenyl]-7-hydroxythiazolo[5,4-d]pyrimidine |
| Compound 7 | 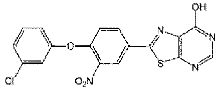 | 2-[4-(3-Chlorophenoxy)-3-nitrophenyl]-7-hydroxythiazolo[5,4-d]pyrimidine |
| Compound 8 | 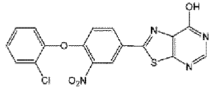 | 2-[4-(2-Chlorophenoxy)-3-nitrophenyl]-7-hydroxythiazolo[5,4-d]pyrimidine |
| Compound 9 | 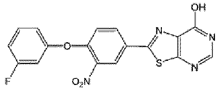 | 2-[4-(3-Fluorophenoxy)-3-nitrophenyl]-7-hydroxythiazolo[5,4-d]pyrimidine |
| Compound 10 | 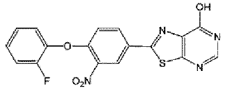 | 2-[4-(2-Fluorophenoxy)-3-nitrophenyl]-7-hydroxythiazolo[5,4-d]pyrimidine |
| Compound 11 | 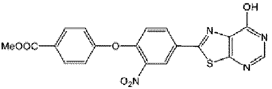 | 7-Hydroxy-2-[4-(4-methoxycarbonylphenoxy)-3-nitrophenyl]thiazolo[5,4-d]pyrimidine |
| Compound 12 | 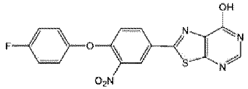 | 2-[4-(4-Fluorophenoxy)-3-nitrophenyl]-7-hydroxyoxazolo[5,4-d]pyrimidine |
| Compound 13 | 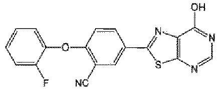 | 2-[3-Cyano-4-(2-fluorophenoxy)phenyl]-7-hydroxythiazolo[5,4-d]pyrimidine |
| Compound 14 | 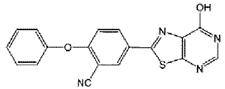 | 2-(3-Cyano-4-phenoxyphenyl)-7-hydroxythiazolo[5,4-d]pyrimidine |
| Compound 15 | 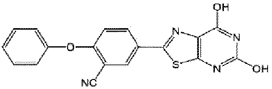 | 5-(5,7-Dihydroxythiazolo[5,4-d]pyrimidin-2-yl)-2-phenoxybenzonitrile |

Pharmaceutically acceptable salts may be formed from Compounds 1 to 15, and among these, Compounds 3 to 5, Compounds 8 to 10, Compound 13, Compound 14, or a pharmaceutically acceptable salt of these compounds is preferable.

It is preferable that the compound represented by General Formula (I) or a pharmaceutically acceptable salt thereof is partially or entirely amorphous in the pharmaceutical composition of the present invention for treating or preventing gout or hyperuricemia. Here, the term "amorphous" means that a substance has a form having a short-distance order between atoms or molecules of the compound represented by General Formula (I) or a pharmaceutically acceptable salt thereof and having no long-distance order such as in crystals.

In the present invention, an amorphous state can be identified according to a halo peak shown in X-ray diffraction.

In the present invention, with respect to the total weight of the compound represented by General Formula (I) or a pharmaceutically acceptable salt thereof, an amorphous content is preferably 50 weight% or more, more preferably 80 weight% or more, still more preferably 90 weight% or more, and yet more preferably 95 weight% or more, and an amorphous content may be 100 weight%. Furthermore, the pharmaceutically acceptable salt of the compound represented by General Formula (I) may be crystalline, and in this case, with respect to the total weight, less than 50 weight% may be amorphous, 40 weight% or less may be amorphous, 30 weight% or less may be amorphous, 20 weight% or less may be amorphous, or 10 weight% may be amorphous, or the pharmaceutically acceptable salt of the compound represented by General Formula (I) may not be amorphous at all. The amorphous content can be obtained by an X-ray diffraction method. Regarding the amorphous content, the remainder is crystalline. That is, in the description of each content, the total of the amorphous content and the crystalline content is 100 weight%.

A method, not falling within the scope of the claimed subject-matter, for producing the amorphous compound represented by General Formula (I) or a pharmaceutically acceptable salt thereof can be, for example, subjecting the compound represented by General Formula (I) or a pharmaceutically acceptable salt thereof to a spray-drying method. More specifically, the compound represented by General Formula (I) or a pharmaceutically acceptable salt thereof and optionally a pharmaceutically acceptable additive are added to a solvent described below to prepare a solution or a suspension, the solution or suspension is finely atomized by centrifugal spraying with a rotating disk or pressure spraying with a pressure nozzle, and the atomized solution or suspension is sprayed into a drying medium (for example, heated air or nitrogen gas), thereby obtaining a powdered amorphous dried component. In the spray-drying method, the temperature of the drying medium is, for example, 50 to 120 °C, and preferably 50 to 90 °C. The drying medium may be caused to flow in a certain direction, and can be caused to flow as an air flow, for example, at 0.1 to 0.6 m³/min.

Examples of the solvent used in the spray-drying method include alcohols including alcohols having 1 to 6 carbon atoms such as methanol, ethanol, 1-propanol, 2-propanol, and tert-butyl alcohol, ethers such as tetrahydrofuran (THF), acetonitrile, and water, and these solvents can be used alone or as a mixed solvent of two or more kinds. Among these, ethanol, tetrahydrofuran, or a mixed solvent of these solvents and water is preferable.

Another method, not falling within the scope of the claimed subject-matter, for producing the amorphous compound represented by General Formula (I) or a pharmaceutically acceptable salt thereof is a freeze-drying method. More specifically, the amorphous compound represented by General Formula (I) or a pharmaceutically acceptable salt thereof can also be produced by dissolving the compound represented by General Formula (I) or a pharmaceutically acceptable salt thereof in a solvent and then freeze-drying the solution.

Examples of the solvent used in the freeze-drying method include alcohols including alcohols having 1 to 6 carbon atoms such as methanol, ethanol, 1-propanol, 2-propanol, and tert-butyl alcohol; ethers such as tetrahydrofuran; nitriles such as acetonitrile; and water, and these solvents can be used alone or as a mixed solvent of two or more kinds.

A particle size of the amorphous compound represented by General Formula (I) or a pharmaceutically acceptable salt thereof is not particularly limited. From the viewpoints of the effect of the invention and formulation, the particle size may be, for example, 20 um or less, and is preferably 1 to 15 µm, more preferably 1 to 10 µm, still more preferably 1.5 to 5 µm, and most preferably 2 to 5 µm, as the volume average particle size (D50).

The volume average particle size (D50) can generally be measured by dispersing a measurement sample in a solvent such as water and ethanol and measuring the particle size distribution by a laser diffraction method. The measurement sample may be dispersed in the solvent by irradiation with ultrasonic waves or the like. The particle size distribution can be measured by a particle size distribution measuring apparatus (for example, Shimadzu laser diffraction type particle size distribution measuring apparatus SALD-2200 or the like). The volume average particle size (D50) can be obtained from the obtained particle size distribution result. In addition, commercially available software (for example, Shimadzu WingSALD-2200 version 1.02 or the like) can be used for data collection and analysis.

A pharmaceutically acceptable additive can be added to the pharmaceutical composition of the present invention as necessary. For example, a required amount of a binding agent, a disintegrating agent, a excipient, a lubricant, or the like can be appropriately combined and added to the composition to produce the pharmaceutical composition of the present invention.

Examples of the binding agent include methyl cellulose, hydroxyethyl cellulose, hydroxypropyl cellulose, hypromellose, polyvinylpyrrolidone, gelatin, agar, alginic acid, sodium alginate, partially saponified polyvinyl alcohol, pullulan, partly pregelatinized starch, dextrin, xanthane gum, and gum arabic powder. These may be used alone or two or more types thereof may be used in combination. Among these, hydroxypropyl cellulose, hypromellose, and polyvinylpyrrolidone are preferable.

Examples of the disintegrating agent include crystalline cellulose, carboxymethyl cellulose (also referred to as carmellose), croscarmellose sodium, carboxymethyl cellulose calcium, low-substituted hydroxypropyl cellulose, crospovidone, hydroxypropyl starch, starch, partly pregelatinized starch, and sodium starch glycolate. These may be used alone or two or more types thereof may be used in combination. Among them, croscarmellose sodium, sodium starch glycolate, and crospovidone are preferable, and crospovidone is more preferable. The amount of the disintegrating agent added is, for example, preferably 5 to 30 weight% and more preferably 5 to 15 weight% with respect to the total weight of particles containing the active ingredient. Furthermore, in a case of being added in a tablet, the amount of the disintegrating agent added is preferably 1 to 10 weight% and more preferably 2 to 6 weight% with respect to the total weight of granules for tableting containing the active ingredient.

The excipient can be added in any of a kneading step, a granulation step, and a post-granulation end step for a pharmaceutical preparation. Examples of the excipient include celluloses such as crystalline cellulose, ethyl cellulose, hydroxypropyl cellulose, low-substituted hydroxypropyl cellulose, and hydroxypropyl methylcellulose (also referred to as hypromellose), starches such as corn starch, potato starch, wheat starch, rice starch, partly pregelatinized starch, and hydroxypropyl starch, sugars such as glucose, lactose, white sugar, refined white sugar, powdered sugar, trehalose, dextran, and dextrin, sugar alcohols such as D-mannitol, xylitol, sorbitol, and erythritol, glycerin fatty acid esters, and inorganic salts such as magnesium aluminometasilicate, synthetic hydrotalcite, anhydrous calcium phosphate, precipitated calcium carbonate, calcium silicate, dibasic calcium phosphate hydrate, and sodium hydrogen carbonate, and crystalline cellulose is preferable.

Examples of the lubricant include stearic acid, sodium stearyl fumarate, magnesium stearate, calcium stearate, sucrose fatty acid ester, polyethylene glycol, light anhydrous silicic acid, hardened oils, glycerin fatty acid esters, and a talc. These may be used alone or two or more types thereof may be used in combination. Among these, sodium stearyl fumarate, magnesium stearate, calcium stearate, and sucrose fatty acid esters are preferable.

Furthermore, recrystallization of the compound represented by General Formula (I) or a pharmaceutically acceptable salt thereof from a supersaturated solution can be suppressed by mixing the compound represented by General Formula (I) or a pharmaceutically acceptable salt thereof with an enteric polymer to be used in the pharmaceutical composition. It is preferable that the compound represented by General Formula (I) or a pharmaceutically acceptable salt thereof and the enteric polymer are uniformly mixed when subjected to mixing. Examples of a weight ratio between the compound represented by General Formula (I) or a pharmaceutically acceptable salt thereof and the enteric polymer can include 1:0.5 to 1:10, and the weight ratio is preferably 1:1 to 1:5, more preferably 1:2 to 1:5, and still more preferably 1:1 to 1:4.

Examples of the enteric polymer include the enteric polymer for coating, and a cellulose-based polymer is preferable, and hydroxypropyl methylcellulose, hydroxypropyl methylcellulose phthalate, and hydroxypropyl methylcellulose acetate succinate are more preferable.

Note that, when the compound represented by the general formula (I) or a pharmaceutically acceptable salt thereof is mixed with the enteric polymer, the pharmaceutically acceptable additive can be appropriately added.

### <Enteric-coated preparation>

The pharmaceutical composition according to the present invention is preferably an enteric-coated preparation.

The "enteric-coated preparation" according to the present invention is a preparation designed to prevent degradation of the active ingredient in the stomach or to release the active ingredient mainly in the small intestine, not it in the stomach. The enteric-coated preparation itself is described in the Japanese Pharmacopoeia. As the enteric-coated preparation, dosage forms such as a tablet, a granular agent, fine granules, and a capsule are known. Examples of a production method for these dosage forms include (i) a method of producing enteric-coated granules by coating the active ingredient or the active ingredient and a pharmaceutically acceptable additive with the enteric polymer and obtaining a tablet, a granular agent, fine granules, or a capsule containing the enteric-coated granules, (ii) a method of producing a tablet, a granular agent, fine granules, or a capsule containing the active ingredient and a pharmaceutically acceptable additive and coating the preparation with the enteric polymer, and (iii) a method of encapsulating the active ingredient or the active ingredient and a pharmaceutically acceptable additive in a hard capsule formed of an enteric base.

That is, examples of the enteric-coated preparation according to the present invention include (i) a tablet, a granular agent, fine granules, or a capsule containing enteric-coated granules obtained by coating the active ingredient or the active ingredient and a pharmaceutically acceptable additive with the enteric polymer, (ii) a tablet, a granular agent, fine granules, or a capsule containing the active ingredient and a pharmaceutically acceptable additive and coated with the enteric polymer, and (iii) a hard capsule obtained by encapsulating the active ingredient or the active ingredient and a pharmaceutically acceptable additive in a hard capsule formed of an enteric base.

The enteric base refers to a base composed of an enteric polymer known per se, and examples of the enteric polymer include those presented below as enteric polymers for coating.

Examples of the enteric polymer for coating used in the present invention include an enteric methacrylic acid copolymer such as a methacrylic acid copolymer L and a methacrylic acid copolymer S (for example, Eudragit (registered trademark) L100 and Eudragit (registered trademark) S100, manufactured by Evonik Industries AG), a methacrylic acid copolymer LD (for example, Eudragit (registered trademark) L100-55 and Eudragit (registered trademark) L30D-55, manufactured by Evonik Industries AG), and a methyl acrylate/methyl methacrylate/methacrylic acid copolymer (for example, Eudragit (registered trademark) FS30D, manufactured by Evonik Industries AG), an enteric cellulose-based polymer such as hypromellose (also referred to as hydroxypropyl methylcellulose), hypromellose acetate succinate (manufactured by Shin-Etsu Chemical Co., Ltd., may be abbreviated as HPMCAS), hypromellose phthalate (manufactured by Shin-Etsu Chemical Co., Ltd., may be abbreviated as HPMCP), carboxymethyl ethylcellulose (manufactured by Freund Corporation, may be abbreviated as CMEC), and cellacefate (also referred to as cellulose acetate phthalate), and an enteric vinyl alcohol-based polymer such as polyvinyl alcohol acetate phthalate (manufactured by Colorcon, Inc.), and the enteric cellulose-based polymer is preferable. Among these, hydroxypropyl methylcellulose, hydroxypropyl methylcellulose phthalate, and hydroxypropyl methylcellulose acetate succinate are preferable.

The enteric-coated granules can be produced according to a known method. For example, the enteric-coated granules can be produced by producing granules by a fluidized granulation method such as rolling fluidized bed granulation and fluidized granulation, a rolling granulation method such as a centrifugal rolling granulation method, or a stirring granulation method, and then coating the granules with an enteric coating liquid and drying the granules.

The enteric coating liquid can be prepared by a method of adding the enteric polymer to a solvent and concentrating the solvent as necessary. Examples of the solvent used for preparing the enteric coating liquid include water, an alcohol-based solvent such as methanol and ethanol, and a mixed liquid thereof. A pharmaceutically acceptable additive such as a binding agent, a plasticizer, a coating base, a surfactant, and a excipient can be appropriately added as necessary. The amount of the solvent is not particularly limited, and the solvent can be used in an amount which is 3 to 10 times the total weight of the dissolved matter (that is, the total weight of the enteric polymer and the pharmaceutically acceptable additive).

The tablet, the granular agent, the fine granules, or the capsule containing the active ingredient and a pharmaceutically acceptable additive and coated with the enteric polymer can be produced by producing a tablet, a granular agent, fine granules, or a capsule containing the active ingredient and the pharmaceutically acceptable additive according to a known method, coating the obtained preparation with the enteric coating liquid, and drying the preparation.

As the hard capsule formed of the enteric base, a commercially available hard capsule can be used. For example, a hard capsule formed of an enteric base containing hydroxypropyl methylcellulose or hydroxypropyl methylcellulose acetate succinate can be used, and more specifically, Vcaps (registered trademark) Enteric (manufactured by Capsugel Belgium NV) or the like can be used.

A pharmaceutically acceptable additive can be added to the enteric-coated preparation according to the present invention as necessary. For example, a required amount of a binding agent, a disintegrating agent, a excipient, a lubricant, or the like can be appropriately combined and added to the enteric-coated preparation to produce the pharmaceutical composition of the present invention. Examples of the "pharmaceutically acceptable additive" can include those presented above as the pharmaceutically acceptable additives for the pharmaceutical composition.

The enteric-coated preparation provided by the present invention can exhibit high in vivo absorbability.

### <Solid dispersion>

The pharmaceutical composition of the present invention may be a pharmaceutical composition further including a solid dispersion containing a hypromellose derivative.

"A hypromellose derivative" according to the present invention represents hypromellose itself (may be abbreviated as HPMC), and an organic acid ester of hypromellose. Hypromellose is called hydroxypropyl methylcellulose, and is a mixed methyl group and hydroxypropyl group ether of cellulose. Examples of an organic acid forming an ester with hypromellose include acetic acid, succinic acid, and phthalic acid. Hypromellose according to the present invention may form an ester with one, two or more organic acids selected from among these organic acids.

Examples of the hypromellose derivative used in the present invention include hypromellose, hypromellose acetate succinate (may be abbreviated as HPMCAS), and hypromellose phthalate (may be abbreviated as HPMCP), and hypromellose acetate succinate or a hypromellose phthalate is preferable.

Examples of the hypromellose according to the present invention include hypromellose in which a substitution proportion per monomer unit is 28 to 30% for a methoxy group and 7 to 12% for a hydroxypropoxy group.

Examples of the hypromellose acetate succinate according to the present invention include hypromellose acetate succinates in which a substitution proportion per monomer unit is 20 to 26% and preferably 21 to 25% for a methoxy group, is 5 to 10% and preferably 5 to 9% for a hydroxypropoxyl group, is 5 to 14% and preferably 7 to 11% for an acetyl group, and is 4 to 18% and preferably 10 to 14% for a succinoyl group. Examples of the hypromellose phthalate according to the present invention include hypromellose phthalate in which a substitution proportion per monomer unit is 18 to 24% for a methoxy group, 5 to 10% for a hydroxypropoxy group, and 21 to 35% for a carboxybenzoyl group.

A content of a methoxy group, a hydroxypropoxy group, an acetyl group, a succinoyl group, a carboxybenzoyl group, or the like in the hypromellose derivative can be measured by a method according to a method of measuring a degree of substitution of hypromellose, hypromellose acetate succinate and hypromellose phthalate defined in the 17th revised Japanese Pharmacopoeia.

A viscosity of the hypromellose derivative according to the present invention is not particularly limited as long as the effect of the present invention is obtained, for example, 2.4 to 204 mPa·s, and preferably 2.4 to 3.6 mPa·s can be mentioned.

A viscosity of the hypromellose derivative according to the present invention can be measured by a method according to a method of measuring a viscosity of hypromellose, hypromellose acetate succinate, and hypromellose phthalate defined in the 17th revised Japanese Pharmacopoeia.

A weight ratio between the compound represented by General Formula (I) or a pharmaceutically acceptable salt thereof and the hypromellose derivative can be appropriately adjusted in a range of 1:0.1 to 1:25. The weight ratio between the compound represented by General Formula (I) or a pharmaceutically acceptable salt thereof and the hypromellose derivative is 1:0.1 to 1:10 in one example, 1:0.1 to 1:4 in another example, 1:1 to 1:10 in still another example, 1:2 to 1:5 in yet another example, and 1:3 to 1:4 in yet another example.

A solid dispersion having a weight ratio between 2-(3-cyano-4-phenoxyphenyl)-7-hydroxythiazolo[5,4-d]pyrimidine or a pharmaceutically acceptable salt thereof and the hypromellose derivative of 1:0.1 to 1:25 in one embodiment of the present invention, 1:0.1 to 1:10 in another embodiment, 1:0.1 to 1:4 in still another embodiment, 1:1 to 1:10 in yet another embodiment, 1:2 to 1:5 in yet another embodiment, and 1:3 to 1:4 in yet another embodiment, can be mentioned.

The term "solid dispersion" refers to a solid system containing at least two components, and a solid composition forming a system in which at least two components are uniformly mixed together. In addition, in the solid dispersion, at least one component is generally dispersed throughout the system.

Therefore, one embodiment of the "solid dispersion" according to the present invention is a solid composition which contains the compound represented by General Formula (I) or a pharmaceutically acceptable salt thereof and the hypromellose derivative, and forms a system in which the compound represented by General Formula (I) or a pharmaceutically acceptable salt thereof and the hypromellose derivative are uniformly mixed together.

Another embodiment of the "solid dispersion" according to the present invention is a solid composition forming a system in which the compound represented by General Formula (I) or a pharmaceutically acceptable salt thereof are dispersed throughout the hypromellose derivative. In this case, the compound represented by General Formula (I) or a pharmaceutically acceptable salt thereof forms a dispersed phase as a dispersoid, and the hypromellose derivative forms a continuous phase as a dispersion medium.

The "solid dispersion" according to the present invention is composed of the compound represented by General Formula (I) or a pharmaceutically acceptable salt thereof, the hypromellose derivative, and optionally a pharmaceutically acceptable additive. Examples of the pharmaceutically acceptable additive optionally contained include the additive selected from among a surfactant, a pH-adjusting agent, sugars and a plasticizer. These can be appropriately combined and added to the solid dispersion according to the present invention in required amounts.

Examples of the surfactant that can be used include a cationic surfactant such as sodium bis-(2-ethylhexyl)sulfosuccinate (sodium docusate), and an alkyltrimethylammonium bromide (for example, cetyltrimethylammonium bromide (cetrimide)), an anionic surfactant such as sodium lauryl sulfate, and a nonionic surfactant such as polyoxyethylene sorbitan (for example, Tween (Tween^{™} 20, 40, 60, 80 or 85)), and a sorbitan fatty acid ester (for example, Span^{™} 20, 40, 60, 80, or 85).

Examples of the pH-adjusting agent that can be used include an acid such as succinic acid, maleic acid, tartaric acid, citric acid, and aspartic acid, and an alkali such as sodium hydroxide, magnesium oxide, silicon dioxide, sodium hydrogen carbonate, and L-arginine.

Examples of the sugar that can be used include lactose, white sugar, glucose, fructose, sucrose, maltose (malt sugar), reduced maltose, maltitol, mannitol, erythritol, sorbitol, and xylitol.

Examples of the plasticizer that can be used include triethyl citrate, polyethylene glycol, and triacetin.

The "solid dispersion" according to the present invention may not contain the pharmaceutically acceptable additive. However, when the pharmaceutically acceptable additive is contained, for example, a weight ratio between the compound represented by General Formula (I) or a pharmaceutically acceptable salt thereof and the surfactant is 1:0.01 to 1:2, more preferably 1:0.02 to 1:1.5, and still more preferably 1:0.03 to 1:1.2, a weight ratio between the compound represented by General Formula (I) or a pharmaceutically acceptable salt thereof and the pH-adjusting agent is 1:0.01 to 1:2, more preferably 1:0.02 to 1:1.5, and still more preferably 1:0.03 to 1:1.2, a weight ratio between the compound represented by General Formula (I) or a pharmaceutically acceptable salt thereof and the sugar is 1:0.02 to 1:20, and more preferably 1:0.15 to 1:10, and a weight ratio between the compound represented by General Formula (I) or a pharmaceutically acceptable salt thereof and the plasticizer is 1:0.02 to 1:20, and more preferably 1:0.15 to 1:10.

In the "solid dispersion" according to the present invention, the pharmaceutically acceptable additive may constitute a dispersed phase or a continuous phase of the solid dispersion.

In the "solid dispersion" according to the present invention, it is preferable that a part or all of the compound represented by General Formula (I) or a pharmaceutically acceptable salt thereof be amorphous.

The solid dispersion according to the present invention can be produced by a method known per se. For example, a mixing and grinding method (a mechano-chemical method), a solvent method, a melting method, a hot melt extrusion method, and the like can be used for the production.

Here, in the mixing and grinding method, the compound represented by General Formula (I) or a pharmaceutically acceptable salt thereof and the hypromellose derivative, and optionally a pharmaceutically acceptable additive are mixed together, and a conventional method using a mixer and a grinding machine such as a ball mill and a hammer mill, can be then performed. The solvent method refers to a method in which the compound represented by General Formula (I) or a pharmaceutically acceptable salt thereof and the hypromellose derivative, and optionally a pharmaceutically acceptable additive are dissolved or suspended in a solvent (an organic solvent, water or a mixed solution thereof), and the solvent is then removed to precipitate a solid dispersion or a solid dispersion is precipitated in the solvent.

The solvent can be removed by a method such as spray methods (can be classified into a fluid bed method, a blowing drying method (also called a spray dry method), a rolling layer method, a stirring method, a supercritical method or the like according to an embodiment), a filtration method, an evaporation method, a freeze-drying method, or the like. A spray method is preferable, and a spray-drying method is particularly preferable among these methods.

Regarding the solvent that can be used to produce the solid dispersion according to the present invention, a pharmaceutically acceptable solvent is preferable. For example, ethanol, methanol, 2-propanol, acetone, 2-butanone, methyl isobutyl ketone, tetrahydrofuran (THF), tetrahydropyran, 1,4-dioxane, diethyl ether, toluene, acetonitrile, methylene chloride, chloroform, methyl acetate, ethyl acetate, butyl acetate, acetic acid, formic acid, N,N-dimethylformamide, N,N-dimethylacetamide, and dimethyl sulfoxide may be used.

It is preferable that the compound represented by General Formula (I) or a pharmaceutically acceptable salt thereof and a pharmaceutically acceptable additive optionally added dissolve in such a solvent.

In the spray-drying method, the solid dispersion can be produced by a method known per se. For example, the compound represented by General Formula (I) or a pharmaceutically acceptable salt thereof and the hypromellose derivative, and optionally a pharmaceutically acceptable additive may be added to the solvent to prepare a solution or suspension, the solution or suspension may be formed into a fine mist by centrifugal spraying using a rotating disk or pressure spraying using a pressure nozzle, and this may be sprayed into a drying medium (heated air or nitrogen gas), and thereby a solid dispersion can be obtained as a powdered dried component.

In the spray-drying method, the temperature of the drying medium is, for example, 50 to 120 °C, and preferably 50 to 90 °C. The drying medium may be caused to flow in a certain direction, and can be caused to flow as an air flow, for example, at 0.1 to 0.6 m³/min.

The precipitation method in the solvent methods is preferably a coprecipitation method, and the compound represented by General Formula (I) or a pharmaceutically acceptable salt thereof and the hypromellose derivative, and optionally a pharmaceutically acceptable additive are dissolved or suspended in a solvent, the dissolved compound (I) or the pharmaceutically acceptable salt, and the hypromellose derivative, and the pharmaceutically acceptable additive optionally added are precipitated by lowering the dissolution concentration by addition of an insoluble solvent, lowering a temperature, or the like, and thereby a solid dispersion can be obtained.

The melting method is a method in which the compound represented by General Formula (I) or a pharmaceutically acceptable salt thereof and the hypromellose derivative, and optionally a pharmaceutically acceptable additive are heated to the melting point or the softening point of hypromellose derivatives or higher and stirred, and thus the compound represented by General Formula (I) or the pharmaceutically acceptable salt thereof and the pharmaceutically acceptable additive optionally added are dissolved or dispersed in the hypromellose derivative and then rapidly cooled. In this case, optionally an additive, for example, a plasticizer such as triethyl citrate, polyethylene glycol, and triacetin, and a surfactant can be additionally added. The production can be performed using a stirring granulator having a heating device.

The hot melt extrusion method is a method in which the compound represented by General Formula (I) or a pharmaceutically acceptable salt thereof and the hypromellose derivative, and optionally a pharmaceutically acceptable additive are heated and mixed under pressure using an extruder having a heating device, for example, a 2-screw extruder, and thereby a solid dispersion is obtained. The obtained plastic-like solid dispersion can be ground using a grinding machine to obtain a solid dispersion powder.

The solid dispersion according to the present invention produced according to the above production method can be formed into solid dispersion particles having a certain particle size by a known method, and the solid dispersion particles can be directly used as a powder or a granular agent.

A pharmaceutical composition containing the solid dispersion according to the present invention contains the solid dispersion and a pharmaceutically acceptable additive. Regarding the pharmaceutically acceptable additive, for example, a binding agent, a disintegrating agent, a excipient, a lubricant, and the like are appropriately combined and added in required amounts, and thereby a pharmaceutical composition of the present invention can be produced. Examples of the "pharmaceutically acceptable additive" can include those presented above as the pharmaceutically acceptable additives for the pharmaceutical composition.

The pharmaceutical composition containing the solid dispersion according to the present invention is subjected to a formulation process known per se and formulated into and provided as a solid preparation such as a tablet, a capsule, a granular agent, and a powder or a liquid preparation such as an injection product. Here, regarding the injection product, the pharmaceutical composition of the present invention may be provided as a solid preparation, put into the injection product for use, and used.

Since the present invention has an effect of preventing tableting failures, it is particularly preferable when the solid preparation has a tablet form. In addition, such a solid preparation can be optionally coated.

A content of the solid dispersion in the pharmaceutical composition according to the present invention containing the solid dispersion is 10 to 95 weight%, preferably 30 to 90 weight%, and more preferably 60 to 85 weight% with respect to the total weight of the pharmaceutical composition.

The solid dispersion provided by the present invention can exhibit high in vivo absorbability and storage stability.

The pharmaceutical composition provided by the present invention can be administered orally or parenterally, more preferably orally, to a patient in need of the treatment or the prevention, more specifically to a human or other mammals. Preferably, the pharmaceutical composition provided by the present invention is administered to a human. The pharmaceutical composition provided by the present invention is useful as a drug for treating or preventing hyperuricemia or gout.

One embodiment of the present invention is a pharmaceutical composition for treating or preventing gout or hyperuricemia containing enteric-coated granules obtained by coating the compound represented by General Formula (I) or a pharmaceutically acceptable salt thereof with an enteric polymer, in which the pharmaceutical composition is used so that the compound represented by General Formula (I) or a pharmaceutically acceptable salt thereof is orally administered to a patient in need of the treatment or the prevention in a daily amount of 10 to 320 mg, preferably 20 to 320 mg, more preferably 40 to 280 mg, still more preferably 40 to 240 mg, yet more preferably 40 to 180 mg, yet more preferably 60 to 140 mg, and yet more preferably 60 to 120 mg, and the oral administration is continued for at least 7 days.

Another embodiment of the present invention is a tablet, a granular agent, fine granules, or a capsule for treating or preventing gout or hyperuricemia containing enteric-coated granules obtained by coating the compound represented by General Formula (I) or a pharmaceutically acceptable salt thereof with an enteric polymer, in which the tablet, the granular, the fine granules, or the capsule is used so that the compound represented by General Formula (I) or a pharmaceutically acceptable salt thereof is orally administered to a patient in need of the treatment or the prevention in a daily amount of 10 to 320 mg, preferably 20 to 320 mg, more preferably 40 to 280 mg, still more preferably 40 to 240 mg, yet more preferably 40 to 180 mg, yet more preferably 60 to 140 mg, and yet more preferably 60 to 120 mg, and the oral administration is continued for at least 7 days.

Still another embodiment of the present invention is a pharmaceutical composition for treating or preventing gout or hyperuricemia containing enteric-coated granules obtained by coating the compound represented by General Formula (I) or a pharmaceutically acceptable salt thereof with an enteric polymer, in which the compound represented by General Formula (I) or a pharmaceutically acceptable salt thereof in the enteric-coated granules is further mixed with the enteric polymer, and the pharmaceutical composition is used so that the compound represented by General Formula (I) or a pharmaceutically acceptable salt thereof is orally administered to a patient in need of the treatment or the prevention in a daily amount of 10 to 320 mg, preferably 20 to 320 mg, more preferably 40 to 280 mg, still more preferably 40 to 240 mg, yet more preferably 40 to 180 mg, yet more preferably 60 to 140 mg, and yet more preferably 60 to 120 mg, and the oral administration is continued for at least 7 days.

Yet another embodiment of the present invention is a tablet, a granular agent, fine granules, or a capsule for treating or preventing gout or hyperuricemia containing enteric-coated granules obtained by coating the compound represented by General Formula (I) or a pharmaceutically acceptable salt thereof with an enteric polymer, in which the compound represented by General Formula (I) or a pharmaceutically acceptable salt thereof in the enteric-coated granules is further mixed with the enteric polymer, and the tablet, the granular agent, the fine granules, or the capsule is used so that the compound represented by General Formula (I) or a pharmaceutically acceptable salt thereof is orally administered to a patient in need of the treatment or the prevention in a daily amount of 10 to 320 mg, preferably 20 to 320 mg, more preferably 40 to 280 mg, still more preferably 40 to 240 mg, yet more preferably 40 to 180 mg, yet more preferably 60 to 140 mg, and yet more preferably 60 to 120 mg, and the oral administration is continued for at least 7 days.

Yet another embodiment of the present invention is a pharmaceutical composition for treating or preventing gout or hyperuricemia containing enteric-coated granules obtained by coating the compound represented by General Formula (I) or a pharmaceutically acceptable salt thereof and a pharmaceutically acceptable additive with an enteric polymer, in which the pharmaceutical composition is used so that the compound represented by General Formula (I) or a pharmaceutically acceptable salt thereof is orally administered to a patient in need of the treatment or the prevention in a daily amount of 10 to 320 mg, preferably 20 to 320 mg, more preferably 40 to 280 mg, still more preferably 40 to 240 mg, yet more preferably 40 to 180 mg, yet more preferably 60 to 140 mg, and yet more preferably 60 to 120 mg, and the oral administration is continued for at least 7 days.

Yet another embodiment of the present invention is a tablet, a granular agent, fine granules, or a capsule for treating or preventing gout or hyperuricemia containing enteric-coated granules obtained by coating the compound represented by General Formula (I) or a pharmaceutically acceptable salt thereof and a pharmaceutically acceptable additive with an enteric polymer, in which the tablet, the granular, the fine granules, or the capsule is used so that the compound represented by General Formula (I) or a pharmaceutically acceptable salt thereof is orally administered to a patient in need of the treatment or the prevention in a daily amount of 10 to 320 mg, preferably 20 to 320 mg, more preferably 40 to 280 mg, still more preferably 40 to 240 mg, yet more preferably 40 to 180 mg, yet more preferably 60 to 140 mg, and yet more preferably 60 to 120 mg, and the oral administration is continued for at least 7 days.

Yet another embodiment of the present invention is a pharmaceutical composition for treating or preventing gout or hyperuricemia containing enteric-coated granules obtained by coating the compound represented by General Formula (I) or a pharmaceutically acceptable salt thereof and a pharmaceutically acceptable additive with an enteric polymer, in which the compound represented by General Formula (I) or a pharmaceutically acceptable salt thereof and the pharmaceutically acceptable additive in the enteric-coated granules are further mixed with the enteric polymer, and the pharmaceutical composition is used so that the compound represented by General Formula (I) or a pharmaceutically acceptable salt thereof is orally administered to a patient in need of the treatment or the prevention in a daily amount of 10 to 320 mg, preferably 20 to 320 mg, more preferably 40 to 280 mg, still more preferably 40 to 240 mg, yet more preferably 40 to 180 mg, yet more preferably 60 to 140 mg, and yet more preferably 60 to 120 mg, and the oral administration is continued for at least 7 days.

Yet another embodiment of the present invention is a tablet, a granular agent, fine granules, or a capsule for treating or preventing gout or hyperuricemia containing enteric-coated granules obtained by coating the compound represented by General Formula (I) or a pharmaceutically acceptable salt thereof and a pharmaceutically acceptable additive with an enteric polymer, in which the compound represented by General Formula (I) or a pharmaceutically acceptable salt thereof and the pharmaceutically acceptable additive in the enteric-coated granules are further mixed with the enteric polymer, and the tablet, the granular, the fine granules, or the capsule is used so that the compound represented by General Formula (I) or a pharmaceutically acceptable salt thereof is orally administered to a patient in need of the treatment or the prevention in a daily amount of 10 to 320 mg, preferably 20 to 320 mg, more preferably 40 to 280 mg, still more preferably 40 to 240 mg, yet more preferably 40 to 180 mg, yet more preferably 60 to 140 mg, and yet more preferably 60 to 120 mg, and the oral administration is continued for at least 7 days.

Yet another embodiment of the present invention is a pharmaceutical composition for treating or preventing gout or hyperuricemia containing the compound represented by General Formula (I) or a pharmaceutically acceptable salt thereof and a pharmaceutically acceptable additive and coated with an enteric polymer, in which the pharmaceutical composition is used so that the compound represented by General Formula (I) or a pharmaceutically acceptable salt thereof is orally administered to a patient in need of the treatment or the prevention in a daily amount of 10 to 320 mg, preferably 20 to 320 mg, more preferably 40 to 280 mg, still more preferably 40 to 240 mg, yet more preferably 40 to 180 mg, yet more preferably 60 to 140 mg, and yet more preferably 60 to 120 mg, and the oral administration is continued for at least 7 days.

Yet another embodiment of the present invention is a tablet, a granular agent, fine granules, or a capsule for treating or preventing gout or hyperuricemia containing the compound represented by General Formula (I) or a pharmaceutically acceptable salt thereof and a pharmaceutically acceptable additive and coated with an enteric polymer, in which the tablet, the granular, the fine granules, or the capsule is used so that the compound represented by General Formula (I) or a pharmaceutically acceptable salt thereof is orally administered to a patient in need of the treatment or the prevention in a daily amount of 10 to 320 mg, preferably 20 to 320 mg, more preferably 40 to 280 mg, still more preferably 40 to 240 mg, yet more preferably 40 to 180 mg, yet more preferably 60 to 140 mg, and yet more preferably 60 to 120 mg, and the oral administration is continued for at least 7 days.

Yet another embodiment of the present invention is a pharmaceutical composition for treating or preventing gout or hyperuricemia containing the compound represented by General Formula (I) or a pharmaceutically acceptable salt thereof and a pharmaceutically acceptable additive and coated with an enteric polymer, in which the compound represented by General Formula (I) or a pharmaceutically acceptable salt thereof and the pharmaceutically acceptable additive are further mixed with the enteric polymer, and the pharmaceutical composition is used so that the compound represented by General Formula (I) or a pharmaceutically acceptable salt thereof is orally administered to a patient in need of the treatment or the prevention in a daily amount of 10 to 320 mg, preferably 20 to 320 mg, more preferably 40 to 280 mg, still more preferably 40 to 240 mg, yet more preferably 40 to 180 mg, yet more preferably 60 to 140 mg, and yet more preferably 60 to 120 mg, and the oral administration is continued for at least 7 days.

Yet another embodiment of the present invention is a tablet, a granular agent, fine granules, or a capsule for treating or preventing gout or hyperuricemia containing the compound represented by General Formula (I) or a pharmaceutically acceptable salt thereof and a pharmaceutically acceptable additive and coated with an enteric polymer, in which the compound represented by General Formula (I) or a pharmaceutically acceptable salt thereof and the pharmaceutically acceptable additive are further mixed with the enteric polymer, and the tablet, the granular, the fine granules, or the capsule is used so that the compound represented by General Formula (I) or a pharmaceutically acceptable salt thereof is orally administered to a patient in need of the treatment or the prevention in a daily amount of 10 to 320 mg, preferably 20 to 320 mg, more preferably 40 to 280 mg, still more preferably 40 to 240 mg, yet more preferably 40 to 180 mg, yet more preferably 60 to 140 mg, and yet more preferably 60 to 120 mg, and the oral administration is continued for at least 7 days.

Yet another embodiment of the present invention is a hard capsule for treating or preventing gout or hyperuricemia obtained by encapsulating the compound represented by General Formula (I) or a pharmaceutically acceptable salt thereof in a hard capsule formed of an enteric base, in which the hard capsule is used so that the compound represented by General Formula (I) or a pharmaceutically acceptable salt thereof is orally administered to a patient in need of the treatment or the prevention in a daily amount of 10 to 320 mg, preferably 20 to 320 mg, more preferably 40 to 280 mg, still more preferably 40 to 240 mg, yet more preferably 40 to 180 mg, yet more preferably 60 to 140 mg, and yet more preferably 60 to 120 mg, and the oral administration is continued for at least 7 days.

Yet another embodiment of the present invention is a hard capsule for treating or preventing gout or hyperuricemia obtained by encapsulating the compound represented by General Formula (I) or a pharmaceutically acceptable salt thereof and a pharmaceutically acceptable additive in a hard capsule formed of an enteric base, in which the hard capsule is used so that the compound represented by General Formula (I) or a pharmaceutically acceptable salt thereof is orally administered to a patient in need of the treatment or the prevention in a daily amount of 10 to 320 mg, preferably 20 to 320 mg, more preferably 40 to 280 mg, still more preferably 40 to 240 mg, yet more preferably 40 to 180 mg, yet more preferably 60 to 140 mg, and yet more preferably 60 to 120 mg, and the oral administration is continued for at least 7 days.

Yet another embodiment of the present invention is a hard capsule for treating or preventing gout or hyperuricemia obtained by encapsulating the compound represented by General Formula (I) or a pharmaceutically acceptable salt thereof in a hard capsule formed of an enteric base, in which the compound represented by General Formula (I) or a pharmaceutically acceptable salt thereof is further mixed with the enteric polymer, and the hard capsule is used so that the compound represented by General Formula (I) or a pharmaceutically acceptable salt thereof is orally administered to a patient in need of the treatment or the prevention in a daily amount of 10 to 320 mg, preferably 20 to 320 mg, more preferably 40 to 280 mg, still more preferably 40 to 240 mg, yet more preferably 40 to 180 mg, yet more preferably 60 to 140 mg, and yet more preferably 60 to 120 mg, and the oral administration is continued for at least 7 days.

Yet another embodiment of the present invention is a hard capsule for treating or preventing gout or hyperuricemia obtained by encapsulating the compound represented by General Formula (I) or a pharmaceutically acceptable salt thereof and a pharmaceutically acceptable additive in a hard capsule formed of an enteric base, in which the compound represented by General Formula (I) or a pharmaceutically acceptable salt thereof and the pharmaceutically acceptable additive are further mixed with the enteric polymer, and the hard capsule is used so that the compound represented by General Formula (I) or a pharmaceutically acceptable salt thereof is orally administered to a patient in need of the treatment or the prevention in a daily amount of 10 to 320 mg, preferably 20 to 320 mg, more preferably 40 to 280 mg, still more preferably 40 to 240 mg, yet more preferably 40 to 180 mg, yet more preferably 60 to 140 mg, and yet more preferably 60 to 120 mg, and the oral administration is continued for at least 7 days.

Yet another embodiment of the present invention is the compound represented by General Formula (I) or a pharmaceutically acceptable salt thereof for use in treatment or prevention of gout or hyperuricemia, which is used so that the compound represented by General Formula (I) or a pharmaceutically acceptable salt thereof is orally administered to a patient in need of the treatment or the prevention in a daily amount of 10 to 320 mg, preferably 20 to 320 mg, more preferably 40 to 280 mg, still more preferably 40 to 240 mg, yet more preferably 40 to 180 mg, yet more preferably 60 to 140 mg, and yet more preferably 60 to 120 mg, and the oral administration is continued for at least 7 days.

Yet another embodiment of the present invention is a pharmaceutical composition for use in treatment or prevention of gout or hyperuricemia containing the compound represented by General Formula (I) or a pharmaceutically acceptable salt thereof and a pharmaceutically acceptable additive, in which the pharmaceutical composition is used so that the compound represented by General Formula (I) or a pharmaceutically acceptable salt thereof is orally administered to a patient in need of the treatment or the prevention in a daily amount of 10 to 320 mg, preferably 20 to 320 mg, more preferably 40 to 280 mg, still more preferably 40 to 240 mg, yet more preferably 40 to 180 mg, yet more preferably 60 to 140 mg, and yet more preferably 60 to 120 mg, and the oral administration is continued for at least 7 days.

Yet another embodiment of the present invention is the solid dispersion according to the present invention or a pharmaceutical composition containing the solid dispersion for use in treatment or prevention of gout or hyperuricemia, in which the solid dispersion or the pharmaceutical composition containing the solid dispersion is used so that the compound represented by General Formula (I) or a pharmaceutically acceptable salt thereof is orally administered to a patient in need of the treatment or the prevention in a daily amount of 10 to 320 mg, preferably 20 to 320 mg, more preferably 40 to 280 mg, still more preferably 40 to 240 mg, yet more preferably 40 to 180 mg, yet more preferably 60 to 140 mg, and yet more preferably 60 to 120 mg, and the oral administration is continued for at least 7 days.

Yet another embodiment of the present invention is use of the compound represented by General Formula (I) or a pharmaceutically acceptable salt thereof for production of a drug for treating or preventing gout or hyperuricemia, in which the compound represented by General Formula (I) or a pharmaceutically acceptable salt thereof is used so that the compound represented by General Formula (I) or a pharmaceutically acceptable salt thereof is orally administered to a patient in need of the treatment or the prevention in a daily amount of 10 to 320 mg, preferably 20 to 320 mg, more preferably 40 to 280 mg, still more preferably 40 to 240 mg, yet more preferably 40 to 180 mg, yet more preferably 60 to 140 mg, and yet more preferably 60 to 120 mg, and the oral administration is continued for at least 7 days.

Yet another embodiment of the present invention is a method for treating or preventing gout or hyperuricemia including orally administering the pharmaceutical composition of the present invention to a subject in need of treatment or prevention of gout or hyperuricemia in a daily amount of 10 to 320 mg, preferably 20 to 320 mg, more preferably 40 to 280 mg, still more preferably 40 to 240 mg, yet more preferably 40 to 180 mg, yet more preferably 60 to 140 mg, and yet more preferably 60 to 120 mg, in which the oral administration is continued for at least 7 days.

Yet another embodiment of the present invention is a method for treating or preventing gout or hyperuricemia including orally administering the solid dispersion according to the present invention or a pharmaceutical composition containing the solid dispersion to a subject in need of treatment or prevention of gout or hyperuricemia in a daily amount of 10 to 320 mg, preferably 20 to 320 mg, more preferably 40 to 280 mg, still more preferably 40 to 240 mg, yet more preferably 40 to 180 mg, yet more preferably 60 to 140 mg, and yet more preferably 60 to 120 mg, in which the oral administration is continued for at least 7 days.

The dose of the pharmaceutical composition of the present invention can be adjusted according to the content of the compound represented by General Formula (I) or a pharmaceutically acceptable salt thereof in the pharmaceutical composition of the present invention. In addition, the dose can be appropriately determined according to an administering method, and age, body weight, gender, symptoms and sensitivity to a drug of an administering subject, and the like, but it may be adjusted according to the progress of improvement in symptoms. In the present invention, it is preferable that the pharmaceutical composition is continuously administered in a constant dose.

The enteric-coated preparation of the present invention can be orally administered, for example, to an adult in a daily dose of generally 10 to 320 mg, preferably 20 to 320 mg, more preferably 40 to 280 mg, still more preferably 40 to 240 mg, yet more preferably 40 to 180 mg, yet more preferably 60 to 140 mg, yet more preferably 60 to 120 mg, and particularly preferably 60 to 100 mg in terms of the content of the compound represented by General Formula (I) or a pharmaceutically acceptable salt thereof, and the oral administration can be continued for at least 7 days. However, the dose and the duration of the administration can be changed according to age, symptoms, and the like. In addition, the number of administration is, for example, 1 to 3 times, preferably 1 or 2 times, and more preferably 1 time per day.

Hyperuricemia is a condition in which the blood uric acid level is abnormally high. In hyperuricemia, part of the uric acid cannot be completely dissolved, and the uric acid crystallizes. When the crystallized uric acid accumulates in the joint, resulting in inflammation, gouty arthritis is caused, which causes a symptom known as a gout attack accompanied by severe pain. Hyperuricemia includes hyperuricemia showing such clinical symptom of gouty arthritis and asymptomatic hyperuricemia with no clinical symptoms such as gouty arthritis.

A subject of the administration of the pharmaceutical composition provided by the present invention may be a gouty arthritis patient (for example, a gouty arthritis patient who develops hyperuricemia) or a hyperuricemia patient who has had a gout attack, or even a patient with asymptomatic hyperuricemia. Preferably, the pharmaceutical composition provided by the present invention may be used for the treatment of hyperuricemia in a gouty arthritis patient.

In the present specification, the terms "suppression of a gout attack", "suppression of induction of a gout attack", and "suppression of onset of a gout attack" refer to a decrease in frequency or severity (for example, a degree of pain or the like), preferably the frequency, of a "gout attack" compared to a group of subjects administered with a conventional uric acid-lowering drug, for example, allopurinol or febuxostat, or a placebo group. The terms preferably include the absence of a "gout attack".

Generally, when treating a gouty arthritis patient in order to lower the blood uric acid level, a uric acid-lowering drug is administered after the remission of gout attacks (after the pain has subsided), however, it is required that the administration starts at a low dose, and the dose is gradually increased so that the blood uric acid level is gradually lowered to a target value. This is required since, when the blood uric acid level is rapidly lowered by the administration of the uric acid-lowering drug, the urate crystals attached to the joint are rapidly redissolved, and as a result, peeling easily occurs, and a gout attack is induced. A gout attack caused by the rapid decrease in the uric acid level due to a uric acid-lowering drug is known as a mobilization flare-up.

Since the pharmaceutical composition provided by the present invention can gradually decrease the blood uric acid concentration, acute gout attacks such as mobilization flare-ups can be suppressed without the gradual increases in the dose or with less gradual increases in the dose.

Meanwhile, it is recommended in Japan that the administration of a xanthine oxidase inhibitor febuxostat starts at a dose of 10 mg once daily, and the dose is gradually increased to 20 mg once daily 2 weeks after the start of the administration and to 40 mg once daily 6 weeks after the start of the administration, in order to suppress the induction of gout attacks (mobilization flare-ups) by the rapid lowering of the blood uric acid level.

In one embodiment, the gradual increase of the daily dose for the suppression of the onset of gout attacks (mobilization flare-ups) which accompanies the initiation of a uric acid-lowering therapy is not necessary for the pharmaceutical composition provided by the present invention, and the daily dose may not be increased within 3 weeks from the start of the administration.

In one embodiment, the gradual increase of the daily dose for the suppression of the onset of gout attacks (mobilization flare-ups) which accompanies the initiation of a uric acid-lowering therapy is not necessary for the pharmaceutical composition provided by the present invention, and the daily dose may not be increased or may be increased once or less within 7 weeks from the start of the administration.

The effect of gradually lowering the blood uric acid level which can be achieved by administering the pharmaceutical composition provided by the present invention can be achieved by, for example, oral administration of the compound represented by General Formula (I) or a pharmaceutically acceptable salt thereof in a daily amount of 10 to 320 mg (for example, 10 to 160 mg, 20 to 160 mg, 40 to 160 mg, 80 to 160 mg, 10 to 80 mg, 20 to 80 mg, 40 to 80 mg, 10 mg, 20 mg, 40 mg, 80 mg, 100 mg, 120 mg, 140 mg, or 160 mg). In addition, the blood uric acid level may be gradually lowered by administration of the compound represented by General Formula (I) or a pharmaceutically acceptable salt thereof in a daily amount of 10 to 320 mg (for example, 10 to 160 mg, 20 to 160 mg, 40 to 160 mg, 80 to 160 mg, 10 to 80 mg, 20 to 80 mg, 40 to 80 mg, 10 to 40 mg, 20 to 40 mg, 10 mg, 20 mg, 40 mg, 80 mg, 100 mg, 120 mg, 140 mg, or 160 mg) every day. For example, the blood uric acid level may be gradually lowered by the administration for 7 or more consecutive days (for example, 1 month, 2 months, or 3 months).

In one embodiment, the pharmaceutical composition of the present invention containing the compound represented by General Formula (I) or a pharmaceutically acceptable salt thereof is used so that the compound represented by General Formula (I) or a pharmaceutically acceptable salt thereof is orally administered to a patient in need of the treatment in a daily amount of 10 to 320 mg, and the oral administration is continued for at least 7 days. Such an embodiment can be useful for the treatment of gout or hyperuricemia which reduces the induction of a gout attack or the severity of an attack, as the uric acid level of the patient is gradually lowered instead of being rapidly lowered.

The effect of gradually lowering the blood uric acid level which is achieved by administering the pharmaceutical composition provided by the present invention may be confirmed, for example, when a maximum rate of decrease in a blood uric acid level on the first day of the administration ([(pre-administration uric acid level - minimum post-administration uric acid level on first day of administration)/pre-administration uric acid level] × 100) is about 35% or less (for example, 1 to 35% decrease, 10 to 30% decrease, 10 to 25% decrease, 10 to 20% decrease, or 15 to 25% decrease); and/or the maximum rate of decrease in a blood uric acid level on the seventh day of the administration ([(pre-administration uric acid level - minimum post-administration uric acid level on the seventh day of administration)/pre-administration uric acid level] × 100) is about 55% or less (for example, 1 to 55% decrease, 10 to 50% decrease, 15 to 45% decrease, 20 to 45% decrease, 20 to 40% decrease, or 20 to 35% decrease), the maximum rates of decrease being determined by continuously administering the pharmaceutical composition provided by the present invention to a healthy adult (for example, a male) for 7 days and measuring the blood uric acid levels before and after the administration.

The blood uric acid level (blood uric acid concentration) may be measured by a known uricaseperoxidase method.

In the present specification, the time or period indicated with the expression "from the start of administration" can be determined by, for example, considering the point of the first administration of the compound represented by General Formula (I) or a pharmaceutically acceptable salt thereof as the starting point in one treatment plan. Therefore, for example, "3 weeks of administration after the start of administration" refers to the time corresponding to 3 weeks after the first administration, and "7 weeks of administration after the start of administration" refers to the time corresponding to 7 weeks after the first administration.

The time or period indicated following the term "administration" refers to the point when the indicated time or period has elapsed since the starting point which is the point of the administration. For example, "12 hours of administration" refers to the point when 12 hours have elapsed since the administration, and "7 days of administration" refers to the point when 7 days have elapsed since the administration. In the present specification, the term "period" is usually used for time defined by "day", "month", or "year".

In the present specification, the meaning of the expression "day X of administration" can be determined in context. For example, while "the first day of administration" may refer to the point when one day (24 hours) has elapsed since the first administration in one treatment plan, the expression may also refer to the period up to immediately before the point when one day (24 hours) has elapsed since the first administration, or refer to the time before or after, for example, ± 12 hours, preferably ± 6 hours, and more preferably ± 2 hours after the point when one day (24 hours) has elapsed since the first administration.

In the present specification, the "minimum post-administration uric acid level on the first day of administration" refers to, for example, the minimum uric acid level among the uric acid levels measured according to a method known per se by using blood collected from the administration subject every hour until one day has elapsed (24 hours have elapsed) since the first administration in one treatment plan. In addition, the "minimum post-administration uric acid level on the seventh day of administration" refers to, for example, the minimum uric acid level among the uric acid levels measured according to a method known per se by using blood collected from the administration subject every hour during the period between the point when 6 days have elapsed (144 hours have elapsed) since the first administration and the point when 7 days have elapsed since the first administration in one treatment plan. Regarding the term "pre-administration", in one treatment plan, 12 hours before the first administration is preferable, 6 hours before the first administration is more preferable, 2 hours before the first administration is still more preferable, and 1 hour before the first administration is particularly preferable. Therefore, the "pre-administration uric acid level" refers to, for example, the uric acid level measured according to a method known per se by using blood collected from the administration subject 12 hours before the first administration, preferably 6 hours before the first administration, more preferably 2 hours before the first administration, and still more preferably 1 hour before the first administration in one treatment plan.

The content of the compound represented by General Formula (I) or a pharmaceutically acceptable salt thereof in the pharmaceutical composition provided by the present invention can be appropriately set by those skilled in the art. For example, the content of the compound represented by General Formula (I) or a pharmaceutically acceptable salt thereof in the pharmaceutical composition provided by the present invention can be 10 to 320 mg (for example, 10 to 160 mg, 20 to 160 mg, 40 to 160 mg, 80 to 160 mg, 10 to 80 mg, 20 to 80 mg, 40 to 80 mg, 10 to 40 mg, 20 to 40 mg, 10 mg, 20 mg, 40 mg, 80 mg, 100 mg, 120 mg, 140 mg, or 160 mg) .

The pharmaceutical composition provided by the present invention can be one dosage unit. A content of the compound represented by General Formula (I) or a pharmaceutically acceptable salt thereof per dosage unit can be, for example, 10 to 320 mg (for example, 10 to 160 mg, 20 to 160 mg, 40 to 160 mg, 80 to 160 mg, 10 to 80 mg, 20 to 80 mg, 40 to 80 mg, 10 to 40 mg, 20 to 40 mg, 10 mg, 20 mg, 40 mg, 80 mg, 100 mg, 120 mg, 140 mg, or 160 mg).

A packaging form of the pharmaceutical composition provided by the present invention can be appropriately set by those skilled in the art. Examples of the packaging form include a plastic container, a glass container, a FTP sheet, and the like. The pharmaceutical composition provided by the present invention may be provided as a package containing the pharmaceutical composition, the number of dosage units of the composition being that required for continuous administration for 5 to 15 days. For example, the pharmaceutical composition provided by the present invention may be provided as a PTP sheet containing 5 to 15 (for example, 6, 7, 8, 10, 12, or 14) dosage units.

In the present specification, a "dosage unit" refers to a unit of a preparation, and "one dosage unit" refers to the smallest unit of the preparation. Accordingly, for example, in a case of tablets, the dosage unit is each tablet, and one dosage unit refers to one tablet. In a case of an injection product, the dosage unit is an injection product contained in a sealed container such as an ampule or a vial, and one dosage unit refers to the injection product contained in one sealed container such as an ampule or a vial.

In a case where the pharmaceutical composition provided by the present invention is administered to a human or another mammal, one or two or more dosage units may be administered at a time, or one dosage unit may be divided to be administered.

Examples of another embodiment of the present invention include the following.
<1-1> A pharmaceutical composition for use in treating gout or hyperuricemia, or hyperuricemia in a gouty arthritis patient, the pharmaceutical composition comprising: a compound represented by General Formula (I) or a pharmaceutically acceptable salt thereof, wherein the content of the compound represented by General Formula (I) or a pharmaceutically acceptable salt thereof is 10 mg to 320 mg, the oral administration thereof is continued for at least 7 days, and the patient is a human.
<1-2> The pharmaceutical composition for use according to <1-1>, which suppresses onset of a gout attack accompanying initiation of a uric acid-lowering therapy thereby.
<1-3> The pharmaceutical composition for use according to <1-1> or <1-2>, which is an enteric-coated preparation.
<1-4> The pharmaceutical composition for use according to <1-3>, wherein the enteric-coated preparation is a hard capsule.
<1-5> The pharmaceutical composition for use according to any one of <1-1> to <1-4>, further comprising: a solid dispersion containing a hypromellose, or an organic acid ester of hypromellose.
<1-6> The pharmaceutical composition for use according to <1-5>, wherein the organic acid ester of hypromellose is hypromellose acetate succinate or hypromellose phthalate.
<1-7> The pharmaceutical composition for use according to <1-5> or <1-6>, wherein a weight ratio between the compound represented by General Formula (I) or a pharmaceutically acceptable salt thereof and the hypromellose or the organic acid ester of hypromellose is 1:0.1 to 1:25.
<1-8> The pharmaceutical composition for use according to any one of <1-1> to <1-7>, which is a solid preparation.
<1-9> The pharmaceutical composition for use according to any one of <1-1> to <1-8>, wherein a content of the compound represented by General Formula (I) or a pharmaceutically acceptable salt thereof per dosage unit is 10 mg to 320 mg, 10 mg to 160 mg, 10 mg to 80 mg, or 20 mg to 80 mg.
<1-10> The pharmaceutical composition for use according to <1-9>, wherein a blood uric acid concentration is lowered by 0.5 to 1.5 mg/dL at 12 hours after administration on the first day of the administration as compared with a blood uric acid concentration before the administration; or by continuous administration once/ day for 7 days, a blood uric acid concentration is lowered by 1.5 to 3.0 mg/dL at 12 hours after administration on the seventh day of the administration as compared with the blood uric acid concentration before the administration.
<1-11> The pharmaceutical composition for use according to any one of <1-1> to <1-10>, wherein a daily dose of the compound represented by General Formula (I) or a pharmaceutically acceptable salt thereof is not increased within 3 weeks of the administration after the start of the administration; or is not increased or is increased once within 7 weeks of the administration after the start of the administration.
<1-12> The pharmaceutical composition for use according to <1-11>, wherein a maximum rate of decrease in a blood uric acid level on the first day of the administration, which is calculated by the following formula: [(pre-administration uric acid level - minimum post-administration uric acid level on first day of administration)/pre-administration uric acid level] x100, is 10 to 250; or maximum rate of decrease in a blood uric acid level on the seventh day of the administration, which is calculated by the following formula: [(pre-administration uric acid level - minimum post-administration uric acid level on the seventh day of administration)/pre-administration uric acid level] × 100, is 20 to 45%.
<1-13> The pharmaceutical composition for use according to any one of <1-1> to <1-12>, wherein the compound represented by General Formula (I) or a pharmaceutically acceptable salt thereof is 2-(3-cyano-4-phenoxyphenyl)-7-hydroxythiazolo[5,4-d]pyrimidine or a pharmaceutically acceptable salt thereof.
<1-14> A package comprising: the pharmaceutical composition for use according to any one of <1-1> to <1-13>,
wherein the number of dosage units of the pharmaceutical composition in the package is the number required for continuous administration for 5 to 15 days.

### Examples

Hereinafter, the present invention will be described in more detail with reference to Examples, Reference Examples, Comparative Reference Examples, and Test Examples, but the present invention is not limited thereto.

### <Reference Example 1a and Comparative Reference Example 1a, Production of enteric-coated capsules>

After dissolving 9 g of Compound 14 (2-(3-cyano-4-phenoxyphenyl)-7-hydroxythiazolo[5,4-d]pyrimidine) in 1936 g of a mixed solvent of tetrahydrofuran (may be abbreviated as THF), ethanol, and water (weight ratio:
THF/ethanol/water = 1600.5/254.5/81) (dissolved by slightly heating), the solution was pumped into a spray dryer at a rate of about 5 mL/min via a peristaltic pump and spray dried and granulated from a 2-fluid nozzle (with a diameter of 508 µm) at an inlet temperature of 80°C and an outlet temperature of about 60°C under conditions of a dry air flow of 0.30 m³/min and a nozzle spray air pressure of 1.0 kgf/cm². The obtained dried component was allowed to stand overnight at room temperature to obtain 100% amorphous Compound 14.

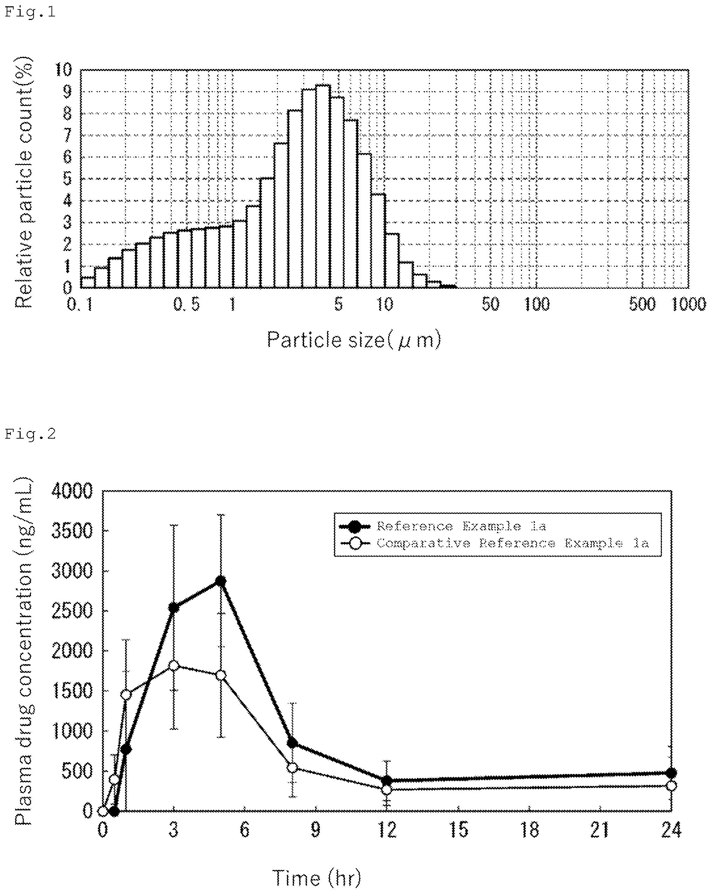

A volume average particle size (D50) of the obtained amorphous Compound 14 was measured with a Shimadzu laser diffraction type particle size distribution measuring device SALD-2200 using a dispersion obtained by adding about 2 mg of a sample to a 0.2% Aerosol OT aqueous solution and irradiating the solution with ultrasonic waves for 30 seconds for dispersion. Shimadzu WingSALD-2200 version 1.02 software was used for data collection and analysis. Figure 1 shows the result of the particle size distribution of amorphous Compound 14. The volume average particle size (D50) of amorphous Compound 14 obtained from the particle size distribution was 2.949 µm.

Each of the capsules shown in Table 2A was filled with 40 mg of the obtained amorphous Compound 14 to obtain capsules of amorphous Compound 14.

**[Table 2A]**

| | Capsule name | Enteric properties | Capsule component | Capsule manufacturer |
|---|---|---|---|---|
| Reference Example 1a | Vcaps (registered trademark) Enteric | Enteric | HPMCAS/HPMC | Lonza/Capsugel |
| Comparative Reference Example 1a | Vcaps Plus (registered trademark) | Not enteric | HPMC | Lonza/Capsugel |

### <Test Example 1a, Absorbability of amorphous Compound 14 in dogs>

Single oral administration of the capsules of Reference Example 1a and Comparative Reference Example 1a (40 mg/body as Compound 14) was performed on beagle dogs (1 to 5 years old, KITAYAMA LABES CO., LTD.) that had been fasted from the evening of the day before the administration one capsule at a time by a crossover method with a one-week administration interval. Blood was collected from the cephalic vein, and the time of the collection was 0.5, 1, 3, 5, 8, and 24 hours before and after the administration. The obtained blood was centrifuged at 10000 ×g and 4°C for 5 minutes to obtain plasma. The concentration of Compound 14 in the plasma was measured by HPLC (OSAKA SODA CO., LTD.). Since dogs generally have a high gastric pH, pentagastrin, a gastric acid stimulator, was intramuscularly injected at a dose of 0.01 mg/kg 30 minutes before and immediately before the administration of the capsules. Then, the pH of a gastric solution was measured to confirm that the gastric pH was low, and the capsule sample was administered.

Plasma drug concentration-time curves were created from the obtained measurement values. The results are shown in Fig. 2. The drug concentrations in the figure show the mean value ± standard deviation of four examples.

When the enteric-coated capsule of Reference Example 1a was used, the drug concentration was higher than that when the general gastric-soluble capsule of Comparative Reference Example 1a, which was not enteric-coated, was used. Thus, absorbability was shown to be improved by the enteric-coating of amorphous Compound 14.

### <Test Example 2a, Evaluation of amorphous state of Compound 14 (powder X-ray diffraction)>

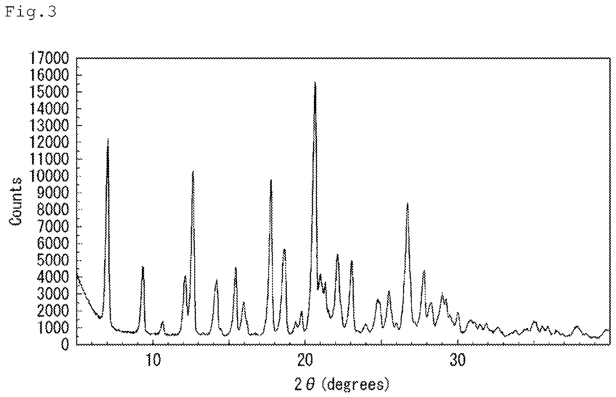

Since it is very important that an amorphous substance retains amorphous properties even after storage over time, amorphous Compound 14 of Reference Example 1a was stored at room temperature in a light-shielded and airtight manner, and a change in the amorphous state of Compound 14 was evaluated using an X-ray diffraction device (D2 Phaser, Bruker). The results are shown in Figs. 4(a) to (d). For comparison, Fig. 3 shows the powder X-ray diffraction of crystalline Compound 14.

When the capsule of Reference Example 1a was stored at room temperature in a light-shielded and airtight manner, changes in the powder X-ray diffraction pattern of Compound 14 over time were not observed during the storage for 4 weeks.

### <Reference Example 1b and Comparative Reference Examples 1b to 9b. Production of solid dispersions (polymer, 25-fold amount)>

250 mg of Compound 14 was dissolved in tetrahydrofuran to obtain a 100 mL of a solution. To 125 mg of each of polymers shown in Table 2B, 5 mL of the mixed solution (dichloromethane/methanol=50/15) was added and dissolved. 2 mL of the solution containing Compound 14 was put into a test tube, and 5 mL of the polymer solution was added thereto and mixed in a vortex mixer to prepare a uniform mixture. In addition, a sample to which no polymer was added was also prepared (Comparative Reference Example 9b). A nitrogen gas stream was blown to this sample, and thereby the solvent was distilled off. Then, drying was performed under a reduced pressure overnight and thereby a solid dispersion sample containing Compound 14 was obtained.

HPMCAS used in Reference Example 1b was of an MF type (specifically, regarding a substitution proportion per monomer unit, a methoxy group: 21.0 to 25.0%, a hydroxypropoxyl group: 5.0 to 9.0%, and an acetyl group: 7.0 to 11.0%, and a succinoyl group: 10.0 to 14.0%, and a viscosity: 2.4 to 3.6 mPa·s).

**[Table 2B]**

| | Polymer | Type | Polymer weight ratio | Manufacturer |
|---|---|---|---|---|
| Reference Example 1b | HPMCAS | MF | 25-Fold amount | Shin-Etsu Chemical Co., Ltd. |
| Comparative Reference Example 1b | HPMC | TC-5 ^{®} | 25-Fold amount | Shin-Etsu Chemical Co., Ltd. |
| Comparative Reference Example 2b | Eudragit | S-100 | 25-Fold amount | Evonik Degussa Japan |
| Comparative Reference Example 3b | Eudragit | E-100 | 25-Fold amount | Evonik Degussa Japan |
| Comparative Reference Example 4b | Eudragit | L-100 | 25-Fold amount | Evonik Degussa Japan |
| Comparative Reference Example 5b | PVP | Kollidon^{®}25 | 25-Fold amount | BASF Japan Ltd. |
| Comparative Reference Example 6b | PEG | 6000 | 25-Fold amount | Wako Pure Chemical Industries, Ltd. |
| Comparative Reference Example 7b | Crospovidone | Kollidon CL-M | 25-Fold amount | BASF Japan Ltd. |
| Comparative Reference Example 8b | Poloxamer 188 | P188 | 25-Fold amount | BASF Japan Ltd. |
| Comparative Reference Example 9b | None | - | - | - |

### <Test Example 1b. Solubility test>

5 mL of a first fluid (pH 1.2) or 5 mL of a second fluid (pH 6.8) for an elution test in Japanese Pharmacopoeia was added to the solid dispersions of Reference Example 1b and Comparative Reference Examples 1b to 8b and the sample of Comparative Reference Example 9b, and the solid dispersions were ground using a glass rod or a spatula, and then shaking was performed at 37°C for 2 hours. 400 µL of a mixed solution (acetonitrile/water=3/2) was immediately added to 600 µL of the sample solution filtered off using a 0.45 um filter, and a concentration of Compound 14 in the sample solution was measured by HPLC (Shimadzu Corporation). The results are shown in Table 3B. Here, values in the table are the mean values obtained by repeating measurements twice.

**[Table 3B]**

| | Sample | Solubility of Compound 14 (µg/mL) |
|---|---|---|
| First fluid for elution test | Comparative Reference Example 3b | 121 |
| | Comparative Reference Example 9b | 0.39 |
| Second fluid for elution test | Reference Example 1b | 44.6 |
| | Comparative Reference Example 4b | 7.32 |
| | Comparative Reference Example 9b | 0.49 |

The solubility of Compound 14 in Comparative Reference Example 9b was 0.39 ug/mL in the first fluid and 0.49 µg/mL in the second fluid. However, in Reference Example 1b and Comparative Reference Examples 3b and 4b, the solubility increased to 5.0 ug/mL or more. In particular, Eudragit E-100 as a basic polymer in Comparative Reference Example 3b in the first fluid under acidic conditions, and HPMCAS as an acidic polymer in Reference Example 1b in the second fluid under neutral conditions had a strong effect of improving the solubility of Compound 14.

### <Reference Example 2b and Comparative Reference Examples 10b and 11b. Production of solid dispersions (polymer, 25-fold amount)>

Compound 14 was dissolved in tetrahydrofuran to prepare a solution having a concentration of 2.5 mg/mL. Each of polymers shown in Table 4B was dissolved in a mixed solvent (methanol/dichloromethane=3/4) to prepare a solution having a concentration of about 45 mg/mL. The solution containing Compound 14 was added to the polymer solution while stirring so that a weight ratio between Compound 14 and the polymer was 1:25. In addition, a sample to which no polymer was added was also prepared. The solution was immediately transferred into an eggplant flask, and the organic solvent was distilled off using a rotary evaporator (N-1100, Tokyo Rikakikai Co., Ltd.). The eggplant flask was transferred to a desiccator and dried under a reduced pressure for about 16 hours using a vacuum pump, and thereby a solid dispersion containing Compound 14 was obtained. The solid dispersion was dried, then ground using an agate mortar or ground using a portable high-speed grinder (LM-PLUS, Osaka Chemical Co., Ltd.), and then sieved (a sieve opening: 150 µm).

**[Table 4B]**

| | Polymer | Type | Polymer weight ratio | Manufacturer |
|---|---|---|---|---|
| Reference Example 2b | HPMCAS | MF | 25-Fold amount | Shin-Etsu Chemical Co., Ltd. |
| Comparative Reference Example 10b | Eudragit | E-100 | 25-Fold amount | Evonik Degussa Japan |
| Comparative Reference Example 11b | None | - | - | - |

### <Test Example 2b. Absorbability of solid dispersion containing Compound 14 in rats>

A single dose of 10 mg/kg or 30 mg/kg, as Compound 14, of the solid dispersions of Reference Example 2b and Comparative Reference Example 10b and the sample of Comparative Reference Example 11b suspended in a 1% carboxymethylcellulose aqueous solution was orally administered to fasted male rats (8 weeks old, Crl:CD (SD), Japan Charles River Laboratories). A crystalline drug substance of Compound 14 was used as a control (Comparative Reference Example 12b). A collection time was 0.5, 1, 2, 4, 8, and 24 hours after administration, and about 300 µL of blood was collected at each time point from vena caudalis (n=3). The obtained blood was centrifuged at 1500×g, 4 °C for 15 minutes to obtain a plasma. The concentration of Compound 14 in the plasma was measured by HPLC (Shiseido Company, Limited and Hitachi High-Technologies Corporation). A time to reach the highest concentration in the plasma (Tₘₐₓ), the highest concentration in the plasma (Cₘₐₓ) and an area under the plasma concentration-time curve (AUC) were calculated from the change in the concentration in the obtained plasma. The results are shown in Table 5B. Here, values in the table are the mean value ± standard deviation of three examples.

The solid dispersions of Reference Example 2b and Comparative Reference Example 10b and the sample of Comparative Reference Example 11b exhibited higher absorption-improving effect on Cₘₐₓ and AUC than those of the drug substance. Therefore, when Compound 14 was prepared in an amorphous form and in a solid dispersion form, improvement in the absorbability was exhibited. In addition, the solid dispersion with HPMCAS exhibited a stronger absorption-improving effect than the solid dispersion with Eudragit.

**[Table 5B]**

| | Dose (mg/kg) | Tₘₐₓ (hr) | Cₘₐₓ (ng/mL) | AUCₗₐₛₜ (ng·hr/mL) |
|---|---|---|---|---|
| Reference Example 2b | 10 | 2.7 ± 1.2 | 3010 ± 228 | 16416 ± 2792 |
| Reference Example 2b | 30 | 4.0 ± 3.5 | 5373 ± 587 | 63160 ± 27388 |
| Comparative Reference Example 10b | 10 | 4.0 ± 0.0 | 1936 ± 1246 | 9422 ± 5470 |
| Comparative Reference Example 10b | 30 | 8.0 ± 0.0 | 1611 ± 284 | 15120 ± 12187 |
| Comparative Reference Example 11b | 30 | 3.7 ± 3.8 | 1724 ± 65 | 15369 ± 7776 |
| Comparative Reference Example 12b | 30 | 4.7 ± 3.1 | 239 ± 99 | 1157 ± 261 |

| | | | | |
|---|---|---|---|---|
| AUCₗₐₛₜ: Area under plasma concentration-time curve up to final observation point | | | | |

### <Reference Examples 3b, 4b, and 5b. Production of solid dispersions (polymer, 25-fold amount)>

Compound 14 was dissolved in tetrahydrofuran to prepare a solution having a concentration of 2.5 mg/mL. Each of polymers shown in Table 6B were dissolved in a mixed solvent (methanol/dichloromethane=3/4) to prepare a solution having a concentration of about 45 mg/mL. Mixing was performed so that a weight ratio between Compound 14 and the polymer was 1:25, and then the solvent was distilled off using a rotary evaporator (N-1100, Tokyo Rikakikai Co., Ltd.) under a reduced pressure at about 50°C. The obtained primary dried component was additionally subjected to secondary drying (room temperature/overnight) using a vacuum pump, and the secondary dried component was appropriately ground using a portable high-speed grinder (LM-PLUS, Osaka Chemical Co., Ltd.) and then sieved (sieve opening: 300 µm).

HPMCAS used in Reference Example 3b was of an LG type (specifically, regarding a substitution proportion per monomer unit, a methoxy group: 20.0 to 24.0%, a hydroxypropoxyl group: 5.0 to 9.0%, an acetyl group: 5.0 to 9.0%, and a succinoyl group: 14.0 to 18.0%, and a viscosity: 2.4 to 3.6 mPa·s).

HPMCAS used in Reference Example 4b was of an MG type (specifically, regarding a substitution proportion per monomer unit, a methoxy group: 21.0 to 25.0%, a hydroxypropoxyl group: 5.0 to 9.0%, an acetyl group: 7.0 to 11.0%, and a succinoyl group: 10.0 to 14.0%, and a viscosity: 2.4 to 3.6 mPa·s).

HPMCAS used in Reference Example 5b was of an HG type (specifically, regarding a substitution proportion per monomer unit, a methoxy group: 22.0 to 26.0%, a hydroxypropoxyl group: 6.0 to 10.0%, an acetyl group: 10.0 to 14.0%, and a succinoyl group: 4.0 to 8.0%, and a viscosity: 2.4 to 3.6 mPa·s).

**[Table 6B]**

| | Polymer | Type | Polymer weight ratio | Manufacturer |
|---|---|---|---|---|
| Reference Example 3b | HPMCAS | LG | 25-Fold amount | Shin-Etsu Chemical Co., Ltd. |
| Reference Example 4b | HPMCAS | MG | 25-Fold amount | Shin-Etsu Chemical Co., Ltd. |
| Reference Example 5b | HPMCAS | HG | 25-Fold amount | Shin-Etsu Chemical Co., Ltd. |

### <Test Example 3b. Absorbability of solid dispersion containing Compound 14 in rats>

A single dose of 10 mg/kg, as Compound 14, of the solid dispersions of Reference Examples 3b, 4b, and 5b suspended in a 1% carboxymethylcellulose aqueous solution was orally administered to fasted male rats (7 to 9 weeks old, Crl:CD (SD), Japan Charles River Laboratories). A collection time was 0.5, 1, 2, 4, 8, and 24 hours after administration, and about 300 µL of blood was collected at each time point from vena caudalis (n=3). The obtained blood was centrifuged at 1500×g, 4 °C for 15 minutes to obtain a plasma. The concentration of Compound 14 in the plasma was measured by HPLC (Shiseido Company, Limited and Hitachi High-Technologies Corporation). A time to reach the highest concentration in the plasma (Tₘₐₓ), the highest concentration in the plasma (Cₘₐₓ) and an area under the plasma concentration-time curve (AUC) were calculated from the change in the concentration in the obtained plasma. The results are shown in Table 7B. Here, values in the table are the mean value ± standard deviation of three examples.

In the solid dispersion of Reference Example 4b, Cₘₐₓ and AUC exhibited the highest concentration in the plasma.

**[Table 7B]**

| | Type | Dose (mg/kg) | Tₘₐₓ (hr) | Cₘₐₓ (ng/mL) | AUCₗₐₛₜ (ng·hr/mL) |
|---|---|---|---|---|---|
| Reference Example 3b | LG | 10 | 3.3 ± 1.2 | 3089 ± 532 | 16342 ± 2494 |
| Reference Example 4b | MG | 10 | 3.3 ± 1.2 | 3961 ± 943 | 24039 ± 6189 |
| Reference Example 5b | HG | 10 | 2.3 ± 1.5 | 1836 ± 112 | 8970 ± 2058 |

| | | | | | |
|---|---|---|---|---|---|
| AUCₗₐₛₜ: Area under plasma concentration-time curve up to final observation point | | | | | |

### <Reference Examples 6b to 12b. Production of solid dispersions (HPMCAS-MG, 1- to 10-fold amounts)>

Compound 14 was dissolved in tetrahydrofuran to prepare a solution having a concentration of 2.5 mg/mL. A HPMCAS-MG was dissolved in a mixed solvent (ethanol/water=4/1) and adjusted to about 45 mg/mL. Mixing was performed so that a weight ratio between Compound 14 and the polymer shown in Table 8B was 1:1 to 1:10. Then, the mixture was pumped into a spray dryer (GB22, Yamato Scientific Co., Ltd.) at a rate of about 5 mL/min via a peristaltic pump, and spray drying and granulation were started from a 2-fluid nozzle (with a diameter of 406 or 508 µm) at an inlet temperature of 80°C and an outlet temperature of about 60°C under conditions of a dry air flow of 0.32 to 0.47 m³/min and a nozzle spray air pressure of 1.0 to 3.1 kgf/cm². The obtained primary dried component was additionally subjected to secondary drying (room temperature/overnight or room temperature/overnight at 40 °C/day) using a vacuum pump and sieved (sieve opening: 300 µm).

**[Table 8B]**

| | Polymer | Type | Polymer weight ratio | Manufacturer |
|---|---|---|---|---|
| Reference Example 6b | HPMCAS | MG | 1-Fold amount | Shin-Etsu Chemical Co., Ltd. |
| Reference Example 7b | HPMCAS | MG | 2-Fold amount | Shin-Etsu Chemical Co., Ltd. |
| Reference Example 8b | HPMCAS | MG | 3-Fold amount | Shin-Etsu Chemical Co., Ltd. |
| Reference Example 9b | HPMCAS | MG | 3.5-Fold amount | Shin-Etsu Chemical Co., Ltd. |
| Reference Example 10b | HPMCAS | MG | 4-Fold amount | Shin-Etsu Chemical Co., Ltd. |
| Reference Example 11b | HPMCAS | MG | 5-Fold amount | Shin-Etsu Chemical Co., Ltd. |
| Reference Example 12b | HPMCAS | MG | 10-Fold amount | Shin-Etsu Chemical Co., Ltd. |

### <Test Example 4b. Absorbability of solid dispersion containing Compound 14 in rats>

A single dose of 10 mg/kg, as Compound 14, of the solid dispersions of Reference Examples 6b to 8b and 10b to 12b suspended in a 1% carboxymethylcellulose aqueous solution was orally administered to fasted male rats (7 to 9 weeks old, Crl:CD (SD), Japan Charles River Laboratories). A collection time was 0.5, 1, 2, 4, 8, and 24 hours after administration, and about 300 µL of blood was collected at each time point from vena caudalis (n=3). The obtained blood was centrifuged at 1500×g, 4 °C for 15 minutes to obtain a plasma. The concentration of Compound 14 in the plasma was measured by HPLC (Shiseido Company, Limited and Hitachi High-Technologies Corporation). A time to reach the highest concentration in the plasma (Tₘₐₓ), the highest concentration in the plasma (Cₘₐₓ) and an area under the plasma concentration-time curve (AUC) were calculated from the change in the concentration in the obtained plasma. The results are shown in Table 9B. Here, values in the table are the mean value ± standard deviation of three examples.

In the solid dispersions of Reference Examples 6b to 8b and 10b to 12b, the concentration in the plasma increased in a weight ratio-dependent manner when weight ratios of HPMCAS-MG with respect to Compound 14 were 1-, 2-, and 3-folds, however, in the range of weight ratios of 4-, 5-, and 10-folds, differences in the concentration in the plasma in a weight ratio-dependent manner were not observed.

**[Table 9B]**

| | Polymer weight ratio | Dose (mg/kg) | Tₘₐₓ (hr) | Cₘₐₓ (ng/mL) | AUCₗₐₛₜ (ng·hr/mL) |
|---|---|---|---|---|---|
| Reference Example 6b | 1-Fold amount | 10 | 2.0 ± 1.7 | 897 ± 579 | 4680 ± 3471 |
| Reference Example 7b | 2-Fold amount | 10 | 4.7 ± 3.1 | 1955 ± 706 | 11029 ± 2832 |
| Reference Example 8b | 3-Fold amount | 10 | 3.3 ± 1.2 | 3970 ± 2048 | 23859 ± 9799 |
| Reference Example 10b | 4-Fold amount | 10 | 2.7 ± 1.2 | 3262 ± 1065 | 17959 ± 3361 |
| Reference Example 11b | 5-Fold amount | 10 | 1.3 ± 0.6 | 3277 ± 604 | 18492 ± 4303 |
| Reference Example 12b | 10-Fold amount | 10 | 2.0 ± 0.0 | 3817 ± 551 | 22612 ± 1610 |

| | | | | | |
|---|---|---|---|---|---|
| AUCₗₐₛₜ: Area under plasma concentration-time curve up to final observation point | | | | | |

### <Test Example 5b. Evaluation of amorphous state of solid dispersion containing Compound 14 (powder X-ray diffraction)>

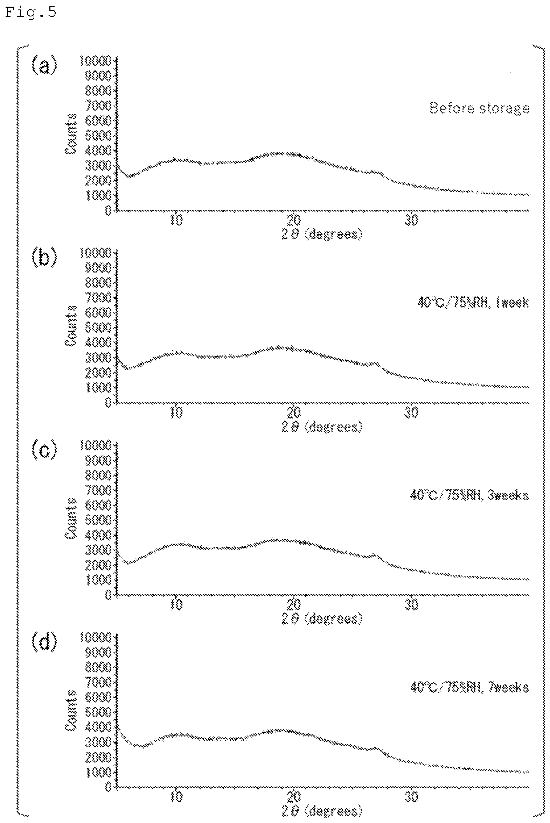

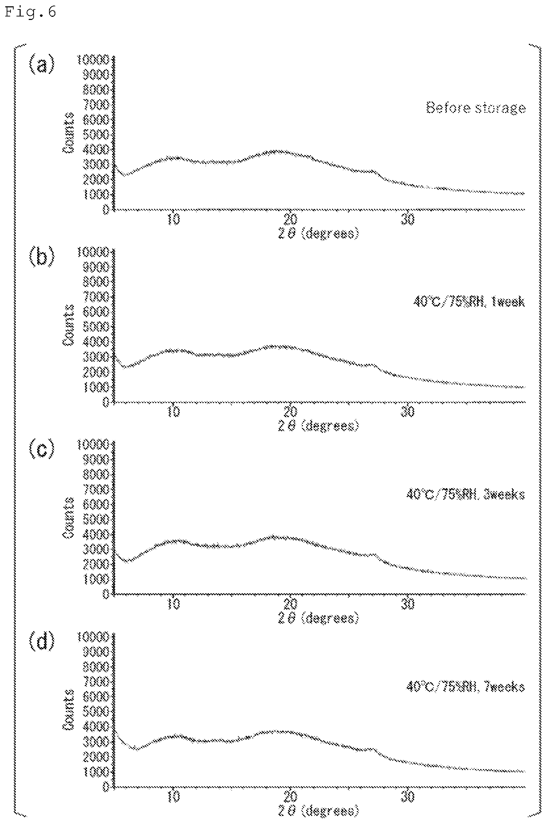

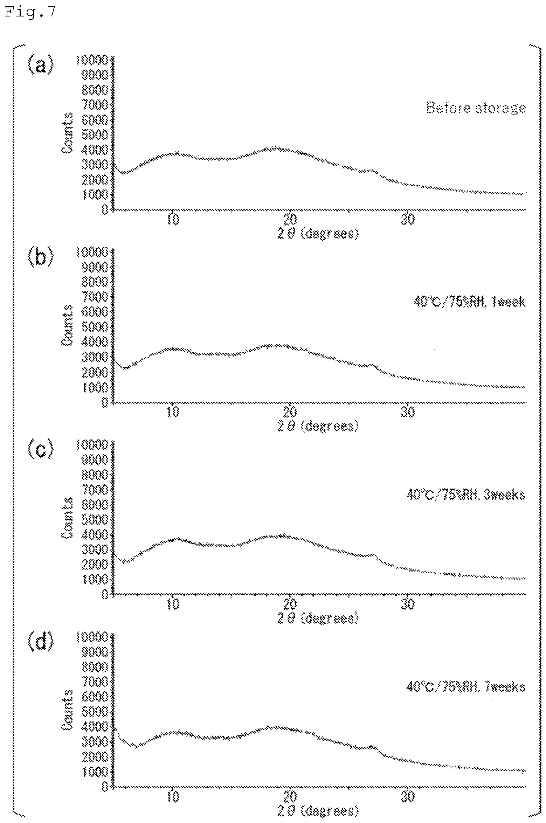

Since it is very important that the solid dispersion retains amorphous properties even after storage over time, the solid dispersions of Reference Examples 9b to 11b were stored under open conditions at 40 °C/75% RH, and a change in the amorphous state was evaluated using an X-ray diffraction device (D2 Phaser, Bruker). The results are shown in Figs. 5 to 7.

When the solid dispersions of Reference Examples 9b to 11b were stored under open conditions at 40 °C/75% RH, changes in the powder X-ray diffraction pattern over time were not observed during the storage for 7 weeks.

### <Test Example 6b. Evaluation of amorphous state of the solid dispersion containing Compound 14 (absorbability in rats)>

The solid dispersions of Reference Examples 9b to 11b were stored under open conditions at 40 °C/75% RH, and a change in the amorphous state was evaluated according to absorbability in rats.

A single dose of 10 or 30 mg/kg, as Compound 14, of the solid dispersions of Reference Examples 9b to 11b suspended in a 1% carboxymethylcellulose aqueous solution was orally administered to fasted male rats (7 to 9 weeks old, Crl:CD (SD), Japan Charles River Laboratories). A collection time was 0.5, 1, 2, 4, 8, and 24 hours after administration, and about 300 µL of blood was collected at each time point from vena caudalis (n=3). The obtained blood was centrifuged at 1500×g, 4 °C for 15 minutes to obtain a plasma. The concentration of Compound 14 in the plasma was measured by HPLC (Shiseido Company, Limited and Hitachi High-Technologies Corporation). A time to reach the highest concentration in the plasma (Tₘₐₓ), the highest concentration in the plasma (Cₘₐₓ) and an area under the plasma concentration-time curve (AUC) were calculated from the change in the concentration in the obtained plasma. The results are shown in Table 10B. Here, values in the table are the mean value ± standard deviation of three examples. When the solid dispersions of Reference Examples 9b to 11b were stored under open conditions at 40 °C/75% RH, decreases in the concentration in the plasma over time were not observed during the storage for 7 weeks.

**[Table 10B]**

| | Dose (mg/kg) | Storage period | Tₘₐₓ (hr) | Cₘₐₓ (ng/mL) | AUCₗₐₛₜ (ng·hr/mL) |
|---|---|---|---|---|---|
| Reference Example 9b | 10 | Before storage | 1.7 ± 0.6 | 3803 ± 679 | 32463 ± 15495 |
| | | 1 week | 3.3 ± 4.0 | 3559 ± 1240 | 18449 ± 4306 |
| | | 3 weeks | 2.7 ± 1.2 | 3265 ± 847 | 19633 ± 5698 |
| | | 7 weeks | 6.7 ± 2.3 | 3570 ± 939 | 15652 ± 5301 |
| | 30 | Before storage | 4.0 ± 3.5 | 4132 ± 633 | 50613 ± 24625 |
| | | 1 week | 6.0 ± 3.5 | 5271 ± 431 | 37881 ± 8970 |
| | | 3 weeks | 4.7 ± 3.1 | 4760 ± 251 | 30196 ± 1575 |
| | | 7 weeks | 6.7 ± 2.3 | 6225 ± 1206 | 70629 ± 19727 |
| Reference Example 10b | 10 | Before storage | 2.3 ± 1.5 | 2870 ± 863 | 23235 ± 4880 |
| | | 1 week | 2.3 ± 1.5 | 3650 ± 914 | 25137 ± 13623 |
| | | 3 weeks | 2.3 ± 1.5 | 3713 ± 1006 | 21674 ± 5359 |
| | | 7 weeks | 3.3 ± 1.2 | 3859 ± 748 | 21209 ± 3935 |
| | 30 | Before storage | 2.7 ± 1.2 | 5798 ± 853 | 50798 ± 8981 |
| | | 1 week | 4.7 ± 3.1 | 6080 ± 1170 | 39819 ± 7414 |
| | | 3 weeks | 6.0 ± 3.5 | 4742 ± 990 | 29954 ± 7461 |
| | | 7 weeks | 4.0 ± 0.0 | 7704 ± 564 | 52920 ± 15388 |
| Reference Example 11b | 10 | Before storage | 6.7 ± 2.3 | 2123 ± 303 | 21294 ± 11194 |
| | | 1 week | 2.3 ± 1.5 | 3217 ± 384 | 19802 ± 2229 |
| | | 3 weeks | 6.7 ± 2.3 | 3687 ± 158 | 19240 ± 9148 |
| | | 7 weeks | 2.7 ± 1.2 | 4833 ± 411 | 24934 ± 854 |
| | 30 | Before storage | 2.7 ± 1.2 | 7557 ± 1529 | 74706 ± 40751 |
| | | 1 week | 3.3 ± 4.0 | 6002 ± 1522 | 29170 ± 10520 |
| | | 3 weeks | 2.0 ± 0.0 | 7504 ± 1978 | 48844 ± 4442 |
| | | 7 weeks | 4.0 ± 3.5 | 6624 ± 360 | 42405 ± 3280 |

| | | | | | |
|---|---|---|---|---|---|
| AUCₗₐₛₜ: Area under plasma concentration-time curve up to final observation point | | | | | |

### <Test Example 7b. Action of solid dispersion containing Compound 14 lowering plasma uric acid level in rats>

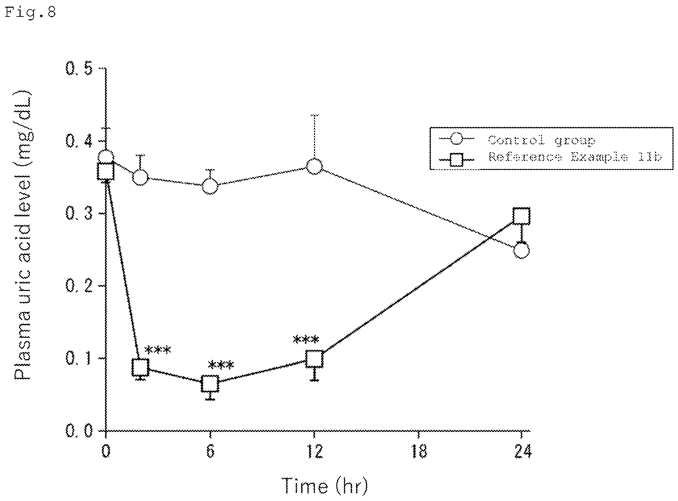

A single dose of 30 mg/kg, as Compound 14, of the solid dispersion of Reference Example 11b suspended in a 1% carboxymethylcellulose aqueous solution was orally administered to fasted male rats (8 weeks old, Crl:CD (SD), Japan Charles River Laboratories). A 1% carboxymethylcellulose aqueous solution was used as a control. A collection time was before administration (0), and 2, 6, 12, and 24 hours after administration, and about 300 µL of blood was collected at each time point from vena caudalis (n=5). The obtained blood was centrifuged at 1500×g, 4 °C for 15 minutes to obtain a plasma. A concentration of uric acid in the plasma was measured by HPLC (Hitachi High-Technologies Corporation). Welch's t-test was performed on plasma uric acid levels at each time point of the control group and Reference Example 11b. A level of significance was p<0.05 (both cases). The results are shown in Fig. 8.

The plasma uric acid level after the solid dispersion of Reference Example 11b was administered was significantly lower than that of the control group, and the action of lowering a uric acid level was persistent.

### <Reference Examples 13b and 14b. Production of solid dispersions (HPMCAS-MG of 3-fold amount and surfactant of 0.03-fold amount)>

Compound 14 was dissolved in tetrahydrofuran to prepare a solution having a concentration of 2.5 mg/mL. A HPMCAS-MG was dissolved in a mixed solvent (ethanol/water=4/1) and adjusted to about 45 mg/mL. Mixing was performed so that a weight ratio among Compound 14 and the polymer and the surfactant (polysorbate 80: Tween 80 and sodium lauryl sulfate: SLS) shown in Table 11B was 1:3:0.03. Then, the mixture was pumped into a spray dryer (GB22, Yamato Scientific Co., Ltd.) at a rate of about 5 mL/min via a peristaltic pump, and spray drying and granulation were started from a 2-fluid nozzle (with a diameter of 406 or 508 µm) at an inlet temperature of 80°C and an outlet temperature of about 60°C under conditions of a dry air flow of 0.32 to 0.47 m³/min and a nozzle spray air pressure of 1.0 to 3.1 kgf/cm². The obtained primary dried component was additionally subjected to secondary drying (room temperature/overnight or room temperature/overnight at 40 °C/day) using a vacuum pump and sieved (sieve opening: 300 µm).

**[Table 11B]**

| | Polymer | Polymer weight ratio | Surfactant | Surfactant weight ratio |
|---|---|---|---|---|
| | Type | Manufacturer | | Manufacturer |
| Reference Example 13b | HPMCAS | 3-Fold amount | Tween 80 | 0.03-Fold amount |
| | MG | Shin-Etsu Chemical Co., Ltd. | | Tokyo Chemical Industry Co., Ltd. |
| Reference Example 14b | HPMCAS | 3-Fold amount | SLS | 0.03-Fold amount |
| | MG | Shin-Etsu Chemical Co., Ltd. | | Kokusan Chemical Co., Ltd. |

### <Test Example 8b. Absorbability of solid dispersion containing Compound 14 in rats>

A single dose of 10 mg/kg, as Compound 14, of the solid dispersions of Reference Examples 13b and 14b suspended in a 1% carboxymethylcellulose aqueous solution was orally administered to fasted male rats (7 to 9 weeks old, Crl:CD (SD), Japan Charles River Laboratories). A collection time was 0.5, 1, 2, 4, 8, and 24 hours after administration, and about 300 µL of blood was collected at each time point from vena caudalis (n=3). The obtained blood was centrifuged at 1500×g, 4 °C for 15 minutes to obtain a plasma. The concentration of Compound 14 in the plasma was measured by HPLC (Shiseido Company, Limited and Hitachi High-Technologies Corporation). A time to reach the highest concentration in the plasma (Tₘₐₓ), the highest concentration in the plasma (Cₘₐₓ) and an area under the plasma concentration-time curve (AUC) were calculated from the change in the concentration in the obtained plasma. The results are shown in Table 12B together with Reference Example 8b. Here, values in the table are the mean value ± standard deviation of three examples.

In the solid dispersions of Reference Examples 13b and 14b, differences were not observed in the concentration in the plasma compared to Reference Example 8b containing no surfactant.

**[Table 12B]**

| | Polymer weight ratio/Surfactant weight ratio | Dose (mg/kg) | Tₘₐₓ (hr) | Cₘₐₓ (ng/mL) | AUCₗₐₛₜ (ng·hr/mL) |
|---|---|---|---|---|---|
| Reference Example 13b | 3-Fold amount/0.03-Fold amount | 10 | 3.3 ± 1.2 | 3625 ± 1335 | 21420 ± 4825 |
| Reference Example 14b | 3-Fold amount/0.03-Fold amount | 10 | 2.7 ± 1.2 | 3589 ± 1370 | 21318 ± 6599 |
| Reference Example 8b | 3-Fold amount | 10 | 3.3 ± 1.2 | 3970 ± 2048 | 23859 ± 9799 |

| | | | | | |
|---|---|---|---|---|---|
| AUCₗₐₛₜ: Area under plasma concentration-time curve up to final observation point | | | | | |

### <Example 1>

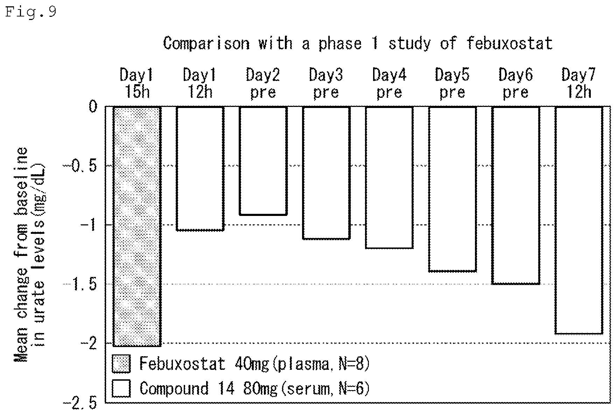

80 mg of Compound 14 was repeatedly administered to 6 healthy adult males once daily for 7 days using solid dispersion capsules of Formulation Example 1, and the action of lowering a serum uric acid level was examined. For comparative reference, the results are shown in Fig. 9 together with the result of a case described in Non Patent Literature 3 in which febuxostat (40 mg) was used.

As a result, with febuxostat, the maximum rate of decrease in a blood uric acid level reached about 35% on the first day of the administration (about 15 hours after the administration), indicating a rapid decrease in the serum uric acid level. On the other hand, the maximum rate of decrease in a blood uric acid level was about 17% on the first day of the administration of Compound 14 (at 12 hours after the administration), and the serum uric acid level was mildly decreased. Thereafter, the serum uric acid level gradually decreased over time throughout the administration period, and the maximum rate of decrease in a blood uric acid level reached about 30% on the seventh day (about 8 hours after the administration).

From these results, Compound 14 is expected to prevent a gout attack or reduce the number of gout attacks.

In addition, as a result of repeated administration of 20 mg of Compound 14 once daily for 7 days using the solid dispersion capsules of Formulation Example 1, the serum uric acid level gradually decreased over time throughout the administration period, which is similar to the result of administering 80 mg of Compound 14 once daily, and 24 hours after the final administration, the serum uric acid level decreased by about 10 to 20% as compared with the serum uric acid level before the administration (initial value).

In addition, as a result of repeated administration of 40 mg of Compound 14 once daily for 7 days using capsules containing micronized Compound 14 (instead of the solid dispersion capsules), the serum uric acid level gradually decreased over time throughout the administration period, which is similar to the results in the cases of using the solid dispersion capsules.

### <Formulation Example 1, Production of solid dispersion capsules>

After dissolving 0.18 kg of Compound 14 (2-(3-cyano-4-phenoxyphenyl)-7-hydroxythiazolo[5,4-d]pyrimidine) in 38.72 kg of a mixed solvent of tetrahydrofuran (may be abbreviated as THF), absolute ethanol, and purified water (weight ratio: 32.01/5.09/1.62) while heating, 0.72 kg of HPMCAS-MG was added thereto and stirred to prepare a feed solution. Then, the feed solution was spray dried with a spray dryer under the conditions of a heat input temperature of 100°C, a heat output temperature of 60°C, a liquid feed rate of 100 g/min, and a 2-fluid nozzle nitrogen spray pressure of 0.30 MPa. The obtained spraydried product was vacuum dried for 16 hours at a drying temperature of 25°C and for 24 hours at 40°C. Then, the vacuum-dried product was air dried for 24 hours at room temperature to obtain a solid dispersion containing Compound 14.

The obtained solid dispersion containing Compound 14 was sieved through a sieve (sieve opening of 300 µm), and then 50 g of sodium starch glycolate was added to and mixed with 250 g of the sieved solid dispersion to obtain a mixed powder of the solid dispersion containing Compound 14.

No. 2 white gelatin capsules were each filled with 60 mg or 120 mg of the obtained mixed powder of the solid dispersion containing Compound 14 to obtain solid dispersion capsules of Compound 14.

### Industrial Applicability

Since the pharmaceutical composition of the present invention containing a compound represented by General Formula (I) or a pharmaceutically acceptable salt thereof gradually decreases the uric acid level of a patient instead of rapidly decreasing it, the pharmaceutical composition can be useful for the treatment or the prevention gout or hyperuricemia which reduces the induction of a gout attack or severity of an attack. The pharmaceutical composition provided by the present invention may be used so that the pharmaceutical composition is orally administered to a patient in need of the treatment or the prevention in an amount of 10 to 320 mg daily, and the oral administration is continued for at least 7 days. In addition, since an enteric-coated preparation of the pharmaceutical composition exhibits high in vivo absorbability, the pharmaceutical composition can be useful for the treatment or the prevention of gout, hyperuricemia. Furthermore, since the pharmaceutical composition exhibits high in vivo absorbability and storage stability when a solid dispersion is contained therein, the pharmaceutical composition can be useful for the treatment or the prevention of gout, hyperuricemia, and the like.

## Claims

1. A pharmaceutical composition for use in treating gout or hyperuricemia, or hyperuricemia in a gouty arthritis patient, the pharmaceutical composition comprising: a compound represented by General Formula (I): 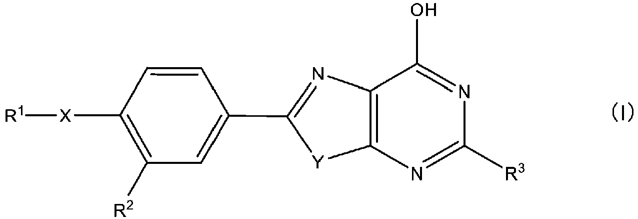
wherein R¹ is an unsubstituted phenyl group or a phenyl group substituted with a substituent, the substituent being at least one group selected from the group consisting of an alkyl group having 1 to 8 carbon atoms, an alkyl group having 1 to 8 carbon atoms substituted with a halogen atom, an alkoxy group having 1 to 8 carbon atoms, an alkoxycarbonyl group having 2 to 8 carbon atoms, a formyl group, a carboxyl group, a halogen atom, a phenyl group, and a phenoxy group, R² is a cyano group or a nitro group, R³ is a hydrogen atom or a hydroxyl group, X is an oxygen atom or -S(O)ₙ-, n is an integer of 0 to 2, and Y is an oxygen atom or a sulfur atom, or a pharmaceutically acceptable salt thereof,
wherein the content of the compound represented by General Formula (I) or a pharmaceutically acceptable salt thereof is 10 mg to 320 mg, the oral administration thereof is continued for at least 7 days, and the patient is a human.

2. The pharmaceutical composition for use according to claim 1, which suppresses onset of a gout attack accompanying initiation of a uric acid-lowering therapy thereby.

3. The pharmaceutical composition for use according to claim 1 or 2, which is an enteric-coated preparation.

4. The pharmaceutical composition for use according to claim 3, wherein the enteric-coated preparation is a hard capsule.

5. The pharmaceutical composition for use according to any one of claims 1 to 4, further comprising: a solid dispersion containing a hypromellose, or an organic acid ester of hypromellose.

6. The pharmaceutical composition for use according to claim 5, wherein the organic acid ester of hypromellose is hypromellose acetate succinate or hypromellose phthalate.

7. The pharmaceutical composition for use according to claim 5 or 6, wherein a weight ratio between the compound represented by General Formula (I) or a pharmaceutically acceptable salt thereof and the hypromellose or the organic acid ester of hypromellose is 1:0.1 to 1:25.

8. The pharmaceutical composition for use according to any one of claims 1 to 7, which is a solid preparation.

9. The pharmaceutical composition for use according to any one of claims 1 to 8, wherein a content of the compound represented by General Formula (I) or a pharmaceutically acceptable salt thereof per dosage unit is 10 mg to 320 mg, 10 mg to 160 mg, 10 mg to 80 mg, or 20 mg to 80 mg.

10. The pharmaceutical composition for use according to claim 9, wherein a blood uric acid concentration is lowered by 0.5 to 1.5 mg/dL at 12 hours after administration on the first day of the administration as compared with a blood uric acid concentration before the administration; or by continuous administration once/day for 7 days, a blood uric acid concentration is lowered by 1.5 to 3.0 mg/dL at 12 hours after administration on the seventh day of the administration as compared with the blood uric acid concentration before the administration.

11. The pharmaceutical composition for use according to any one of claims 1 to 10, wherein a daily dose of the compound represented by General Formula (I) or a pharmaceutically acceptable salt thereof is not increased within 3 weeks of the administration after the start of the administration; or is not increased or is increased once within 7 weeks of the administration after the start of the administration.

12. The pharmaceutical composition for use according to claim 11, wherein a maximum rate of decrease in a blood uric acid level on the first day of the administration, which is calculated by the following formula: [(pre-administration uric acid level - minimum post-administration uric acid level on first day of administration)/pre-administration uric acid level] x 100, is 10 to 25%; or
maximum rate of decrease in a blood uric acid level on the seventh day of the administration, which is calculated by the following formula: [(pre-administration uric acid level - minimum post-administration uric acid level on the seventh day of administration)/pre-administration uric acid level] x 100, is 20 to 45%.

13. The pharmaceutical composition for use according to any one of claims 1 to 12, wherein the compound represented by General Formula (I) or a pharmaceutically acceptable salt thereof is 2-(3-cyano-4-phenoxyphenyl)-7-hydroxythiazolo[5,4-d]pyrimidine or a pharmaceutically acceptable salt thereof.

14. A package comprising: the pharmaceutical composition for use according to any one of claims 1 to 13, wherein the number of dosage units of the pharmaceutical composition in the package is the number required for continuous administration for 5 to 15 days.

## Patentansprüche

1. Pharmazeutische Zusammensetzung zur Verwendung bei der Behandlung von Gicht oder Hyperurikämie oder Hyperurikämie bei einem Gichtarthritis-Patienten, wobei die pharmazeutische Zusammensetzung umfasst: eine Verbindung, dargestellt durch die allgemeine Formel (I): 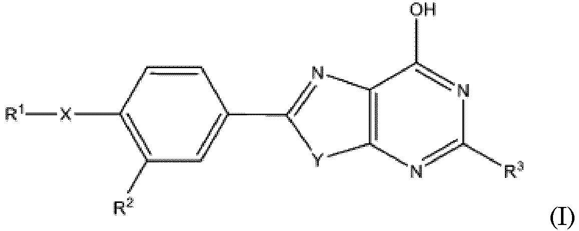
wobei R¹ eine unsubstituierte Phenylgruppe oder eine mit einem Substituenten substituierte Phenylgruppe ist, wobei der Substituent mindestens eine Gruppe ist, ausgewählt aus der Gruppe bestehend aus einer Alkylgruppe mit 1 bis 8 Kohlenstoffatomen, einer Alkylgruppe mit 1 bis 8 Kohlenstoffatomen, substituiert mit einem Halogenatom, einer Alkoxygruppe mit 1 bis 8 Kohlenstoffatomen, einer Alkoxycarbonylgruppe mit 2 bis 8 Kohlenstoffatomen, einer Formylgruppe, einer Carboxylgruppe, einem Halogenatom, einer Phenylgruppe und einer Phenoxygruppe, wobei R² eine Cyanogruppe oder eine Nitrogruppe ist, R³ ein Wasserstoffatom oder eine Hydroxylgruppe ist, X ein Sauerstoffatom oder -S(O)ₙ- ist, wobei n eine ganze Zahl von 0 bis 2 ist und Y ein Sauerstoffatom oder ein Schwefelatom ist, oder ein pharmazeutisch verträgliches Salz davon,
wobei der Gehalt der durch die allgemeine Formel (I) dargestellten Verbindung oder eines pharmazeutisch verträglichen Salzes davon 10 mg bis 320 mg ist, deren orale Verabreichung für mindestens 7 Tage fortgesetzt wird und der Patient ein Mensch ist.

2. Pharmazeutische Zusammensetzung zur Verwendung nach Anspruch 1, die das Auftreten eines Gichtanfalls unterdrückt, der mit dem Beginn einer harnsäuresenkenden Therapie einhergeht.

3. Pharmazeutische Zusammensetzung zur Verwendung nach Anspruch 1 oder 2, die ein magensaftresistentes Präparat ist.

4. Pharmazeutische Zusammensetzung zur Verwendung nach Anspruch 3, wobei das magensaftresistente Präparat eine Hartkapsel ist.

5. Pharmazeutische Zusammensetzung zur Verwendung nach einem der Ansprüche 1 bis 4, ferner umfassend: eine feste Dispersion, enthaltend eine Hypromellose oder einen organischen Säureester von Hypromellose.

6. Pharmazeutische Zusammensetzung zur Verwendung nach Anspruch 5, wobei der organische Säureester von Hypromellose Hypromelloseacetatsuccinat oder Hypromellosephthalat ist.

7. Pharmazeutische Zusammensetzung zur Verwendung nach Anspruch 5 oder 6, wobei das Gewichtsverhältnis zwischen der durch die allgemeine Formel (I) dargestellten Verbindung oder einem pharmazeutisch verträglichen Salz davon und der Hypromellose oder dem organischen Säureester der Hypromellose 1:0,1 bis 1:25 ist.

8. Pharmazeutische Zusammensetzung zur Verwendung nach einem der Ansprüche 1 bis 7, die ein festes Präparat ist.

9. Pharmazeutische Zusammensetzung zur Verwendung nach einem der Ansprüche 1 bis 8, wobei ein Gehalt der durch die allgemeine Formel (I) dargestellten Verbindung oder einem pharmazeutisch verträglichen Salz davon pro Dosierungseinheit 10 mg bis 320 mg, 10 mg bis 160 mg, 10 mg bis 80 mg oder 20 mg bis 80 mg ist

10. Pharmazeutische Zusammensetzung zur Verwendung nach Anspruch 9, wobei eine Harnsäurekonzentration im Blut 12 Stunden nach der Verabreichung am ersten Tag der Verabreichung im Vergleich zu einer Harnsäurekonzentration im Blut vor der Verabreichung um 0,5 bis 1,5 mg/dL gesenkt wird; oder durch kontinuierliche Verabreichung einmal/Tag über 7 Tage eine Harnsäurekonzentration im Blut 12 Stunden nach der Verabreichung am siebten Tag der Verabreichung im Vergleich zu der Harnsäurekonzentration im Blut vor der Verabreichung um 1,5 bis 3,0 mg/dL gesenkt wird.

11. Pharmazeutische Zusammensetzung zur Verwendung nach einem der Ansprüche 1 bis 10, wobei eine tägliche Dosis der durch die allgemeine Formel (I) dargestellten Verbindung oder eines pharmazeutisch verträglichen Salzes davon innerhalb von 3 Wochen nach dem Beginn der Verabreichung nicht erhöht wird; oder innerhalb von 7 Wochen nach dem Beginn der Verabreichung nicht erhöht wird oder einmal erhöht wird.

12. Pharmazeutische Zusammensetzung zur Verwendung nach Anspruch 11, wobei eine maximale Abnahmerate des Harnsäurespiegels im Blut am ersten Tag der Verabreichung, die nach der folgenden Formel berechnet wird: [(Harnsäurespiegel vor der Verabreichung - minimaler (Harnsäurespiegel nach der Verabreichung am ersten Tag der Verabreichung)/Harnsäurespiegel vor der Verabreichung] x 100, 10 bis 25% ist; oder
eine maximale Abnahmerate des Harnsäurespiegels im Blut am siebten Tag der Verabreichung, die nach der folgenden Formel berechnet wird: [(Harnsäurespiegel vor der Verabreichung - minimaler (Harnsäurespiegel nach der Verabreichung am siebten Tag der Verabreichung)/Harnsäurespiegel vor der Verabreichung] x 100, 20 bis 45% ist.

13. Pharmazeutische Zusammensetzung zur Verwendung nach einem der Ansprüche 1 bis 12, wobei die durch die allgemeine Formel (I) dargestellte Verbindung oder ein pharmazeutisch verträgliches Salz davon 2-(3-Cyano-4-phenoxyphenyl)-7-hydroxythiazolo[5,4-d]pyrimidin oder ein pharmazeutisch verträgliches Salz davon ist.

14. Packung, umfassend: die pharmazeutische Zusammensetzung zur Verwendung nach einem der Ansprüche 1 bis 13, wobei die Anzahl der Dosierungseinheiten der pharmazeutischen Zusammensetzung in der Packung der Anzahl entspricht, die für eine kontinuierliche Verabreichung über 5 bis 15 Tage erforderlich ist.

## Revendications

1. Composition pharmaceutique destinée à être utilisée dans le traitement de la goutte ou de l'hyperuricémie, ou de l'hyperuricémie chez un patient souffrant d'arthrite goutteuse, la composition pharmaceutique comprenant : un composé représenté par la formule générale (I) : 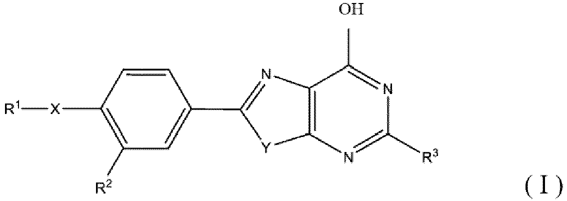
dans laquelle R¹ est un groupe phényle non substitué ou un groupe phényle substitué par un substituant, le substituant étant au moins un groupe choisi dans le groupe constitué d'un groupe alkyle ayant 1 à 8 atomes de carbone, un groupe alkyle ayant 1 à 8 atomes de carbone substitué par un atome d'halogène, un groupe alcoxy ayant 1 à 8 atomes de carbone, un groupe alcoxycarbonyle
ayant 2 à 8 atomes de carbone, un groupe formyle, un groupe carboxyle, un atome d'halogène, un groupe phényle et un groupe phénoxy, R² est un groupe cyano ou un groupe nitro, R³ est un atome d'hydrogène ou un groupe hydroxyle, X est un atome d'oxygène ou -S(O)ₙ-, n est un nombre entier de 0 à 2, et Y est un atome d'oxygène ou un atome de soufre, ou un sel pharmaceutiquement acceptable de celui-ci,
dans laquelle la teneur du composé représenté par la formule générale (I) ou d'un sel pharmaceutiquement acceptable de celui-ci est de 10 mg à 320 mg, l'administration orale de celui-ci est poursuivie pendant au moins 7 jours, et le patient est un être humain.

2. Composition pharmaceutique à utiliser selon la revendication 1, qui supprime l'apparition d'une crise de goutte accompagnant ainsi le début d'un traitement réduisant l'acide urique.

3. Composition pharmaceutique à utiliser selon la revendication 1 ou 2, qui est une préparation à enrobage gastrorésistant.

4. Composition pharmaceutique à utiliser selon la revendication 3, dans laquelle la préparation à enrobage entérique est une capsule dure.

5. Composition pharmaceutique à utiliser selon l'une quelconque des revendications 1 à 4, comprenant en outre : une dispersion solide contenant une hypromellose, ou un ester d'acide organique d'hypromellose.

6. Composition pharmaceutique à utiliser selon la revendication 5, dans laquelle l'ester d'acide organique d'hypromellose est l'acétate succinate d'hypromellose ou le phtalate d'hypromellose.

7. Composition pharmaceutique à utiliser selon la revendication 5 ou 6, dans laquelle un rapport pondéral entre le composé représenté par la formule générale (I) ou un sel pharmaceutiquement acceptable de celui-ci et l'hypromellose ou l'ester d'acide organique de l'hypromellose est de 1:0,1 à 1:25.

8. Composition pharmaceutique à utiliser selon l'une quelconque des revendications 1 à 7, qui est une préparation solide.

9. Composition pharmaceutique à utiliser selon l'une quelconque des revendications 1 à 8, dans laquelle la teneur du composé représenté par la formule générale (I) ou d'un sel pharmaceutiquement acceptable de celui-ci par unité posologique est de 10 mg à 320 mg, de 10 mg à 160 mg, 10 mg à 80 mg ou 20 mg à 80 mg.

10. Composition pharmaceutique à utiliser selon la revendication 9, dans laquelle une concentration sanguine d'acide urique est abaissée de 0,5 à 1,5 mg/dL 12 heures après l'administration le premier jour de l'administration par rapport à une concentration sanguine d'acide urique avant l'administration ;
ou par administration continue une fois/jour pendant 7 jours, la concentration sanguine d'acide urique est abaissée de 1,5 à 3,0 mg/dL 12 heures après l'administration le septième jour de l'administration par rapport à la concentration sanguine d'acide urique avant l'administration.

11. Composition pharmaceutique à utiliser selon l'une quelconque des revendications 1 à 10, dans laquelle une dose quotidienne du composé représenté par la formule générale (I) ou d'un sel pharmaceutiquement acceptable de celui-ci n'est pas augmentée dans les 3 semaines suivant l'administration après le début de l'administration ; ou n'est pas augmentée ou est augmentée une fois dans les 7 semaines suivant l'administration après le début de l'administration.

12. Composition pharmaceutique à utiliser selon la revendication 11, dans laquelle un taux maximum de diminution du taux d'acide urique dans le sang le premier jour de l'administration, qui est calculé par la formule suivante :
[(taux d'acide urique avant l'administration - niveau minimum d'acide urique après l'administration le premier jour d'administration)/taux d'acide urique avant l'administration] x 100, soit 10 à 25 % ; ou taux maximal de diminution du taux d'acide urique dans le sang le septième jour de l'administration, qui est calculé par la formule suivante : [(taux d'acide urique avant l'administration - niveau minimum d'acide urique après l'administration le septième jour d'administration)/taux d'acide urique avant l'administration] x 100, est de 20 à 45 % .

13. Composition pharmaceutique à utiliser selon l'une quelconque des revendications 1 à 12, dans laquelle le composé représenté par la formule générale (I) ou un sel pharmaceutiquement acceptable est la 2-(3-cyano-4-phénoxyphényl)-7-hydroxythiazolo[5,4-d]pyrimidine ou un sel pharmaceutiquement acceptable de celle-ci.

14. Emballage comprenant : la composition pharmaceutique à utiliser selon l'une quelconque des revendications 1 à 13, dans laquelle le nombre d'unités posologiques de la composition pharmaceutique dans l'emballage est le nombre requis pour une administration continue pendant 5 à 15 jours.
